# EUROPEAN PATENT SPECIFICATION

(11) **EP 3 648 789 B1**
(45) Date of publication and mention of the grant of the patent: **07.06.2023**
(21) Application number: 18737584.5
(22) Date of filing: 03.07.2018
(51) Int. Cl.: A61K 38/46, A61K 48/00, A61K 31/7088, A61P 21/00

(54) **MTMR2-S POLYPEPTIDE FOR USE IN THE TREATMENT OF MYOPATHIES**
MTMR2-S POLYPEPTID ZUR VERWENDUNG IN DER BEHANDLUNG VON MYOPATHIEN
POLYPEPTIDE MTMR2-S POUR UTILISATION DANS LE TRAITEMENT DE MYOPATHIES

(30) Priority: 03.07.2017 EP 17305852
(43) Date of publication of application: 13.05.2020
(73) Proprietor: Université de Strasbourg, 67000 Strasbourg (FR); Centre National de la Recherche Scientifique, 75016 Paris (FR); Institut National de la Santé et de la Recherche Médicale, 75013 Paris (FR)
(72) Inventor: LAPORTE, Jocelyn, 67100 Strasbourg (FR); COWLING, Belinda, 67240 Kaltenhouse (FR); RAESS, Matthieu, 67100 Strasbourg (FR); FRIANT-MICHEL, Sylvie, 67380 Lingolsheim (FR); BERTAZZI, Dimitri, 68220 Knoeringue (FR)
(74) Representative: Cabinet Becker et Associés
(86) International application number: PCT/EP2018/068004
(87) International publication number: WO 2019/007991

(56) References cited:
- US-A1- 2015 218 540
- US-A1- 2017 095 540
- ELIZABETH A. FOGARTY ET AL: "SOX10 regulates an alternative promoter at the Charcot-Marie-Tooth disease locus MTMR2", HUMAN MOLECULAR GENETICS, vol. 25, no. 18, 27 July 2016 (2016-07-27) , pages 3925-3936, XP055429963, gb ISSN: 0964-6906, DOI: 10.1093/hmg/ddw233
- N. DANIÈLE ET AL: "P.4.2 MTMR 2 ameliorates the phenotype of myotubular myopathy in mice", NEUROMUSCULAR DISORDERS., vol. 23, no. 9-10, 1 October 2013 (2013-10-01), page 760, XP055429819, GB ISSN: 0960-8966, DOI: 10.1016/j.nmd.2013.06.439
- JAMES J. DOWLING ET AL: "Loss of Myotubularin Function Results in T-Tubule Disorganization in Zebrafish and Human Myotubular Myopathy", PLOS GENETICS, vol. 5, no. 2, 6 February 2009 (2009-02-06), page e1000372, XP055429886, DOI: 10.1371/journal.pgen.1000372
- Anonymous: "MTMR2 - Myotubularin related protein 2, isoform CRA_a - Homo sapiens (Human) - MTMR2 gene & protein", , 9 July 2014 (2014-07-09), XP055429935, Retrieved from the Internet: URL:http://www.uniprot.org/uniprot/A0A024R 3B7 [retrieved on 2017-11-29]

## Description

### FIELD OF THE INVENTION

The present disclosure relates to a MTMR2-S polypeptide, or a nucleic acid sequence producing or encoding said MTMR2-S polypeptide, for a use in the treatment of X-linked centronuclear myopathy (XLCNM), i.e. a disease or disorder associated with MTM1 mutation or deficiency. The present invention provides compositions and methods for treatment of myopathy or diseases or disorders associated with MTM1 mutation or deficiency, in a subject in need thereof. The present invention relates to a method of delivering the MTMR2-S polypeptide to subjects in need of improved muscle function, such as subjects with centronuclear myopathies.

### BACKGROUND OF THE INVENTION

Centronuclear Myopathies (CNM) are a group of congenital myopathies characterized by muscle weakness and confirmed histologically by fiber atrophy, predominance of type I fibers, and increased centralization of nuclei, not secondary to muscle regeneration. Among the three main characterized forms of CNM, X-linked centronuclear myopathy (also called XLCNM, myotubular myopathy - XLMTM, or OMIM 310400) is the most common and severe form of CNM, with neonatal onset and death often occurring in the first years of life (Jungbluth, H. et al., Orphanet J Rare Dis, 2008. 3: p. 26). Survival beyond the postnatal period requires intensive support, often including gastrostomy feeding and mechanical ventilation. There is currently no cure, nor effective treatments available for this disorder.

XLCNM is due to mutations in the phosphoinositides phosphatase myotubularin (MTM1) (Laporte, J. et al., Nature Genetics, 1996. 13(2): p. 175-82). To date more than 200 different mutations in *MTM1* have been reported in about 450 families, most of which lead to a strong reduction of protein. *Mtm*1 knockout or knockin mice have previously been characterized, which recapitulate the CNM phenotype with classical histological features including abnormal organelle positioning, mislocalization of nuclei and muscle atrophy, associated with a corresponding reduction in muscle strength (Buj-Bello A, Laugel V, Messaddeq N, Zahreddine H, Laporte J, Pellissier JF, Mandel JL., The lipid phosphatase myotubularin is essential for skeletal muscle maintenance but not for myogenesis in mice, Proc Natl Acad Sci USA. 2002 Nov 12; 99(23): 15060-5. Epub 2002 Oct 21; Pierson CR, Dulin-Smith AN, Durban AN, Marshall ML, Marshall JT, Snyder AD, Naiyer N, Gladman JT, Chandler DS, Lawlor MW, Buj-Bello A, Dowling JJ, Beggs AH., Hum Mol Genet. 2012 Feb 15;21(4):811-25. doi: 10.1093/hmg/ddr512. Epub 2011 Nov 7; Mol Cell Biol. 2013 Jan;33(1):98-110. doi: 10.1128/MCB.01075-12. Epub 2012 Oct 29.Defective autophagy and mTORC1 signaling in myotubularin null mice. Fetalvero KM, Yu Y, Goetschkes M, Liang G, Valdez RA, Gould T, Triantafellow E, Bergling S, Loureiro J, Eash J, Lin V, Porter JA, Finan PM, Walsh K, Yang Y, Mao X, Murphy LO). A defect in triads structure associated with abnormal excitation-contraction coupling has been detected in several animal models and patients with different forms of CNM, identifying a common defect in all CNM forms (Toussaint A. et al., Acta Neuropathol. 2011 Feb; 121(2):253-66). This is consistent with a proposed role of MTM1 in the regulation of phosphoinositides level on the sarcoplasmic reticulum component of the triads. Loss of phosphatase activity in myotubularin-related protein 2 is associated with Charcot-Marie-Tooth disease type 4B1 (Charcot-Marie-Tooth type 4B is caused by mutations in the gene encoding myotubularin-related protein-2., Bolino A, Muglia M, Conforti FL, LeGuern E, Salih MA, Georgiou DM, Christodoulou K, Hausmanowa-Petrusewicz I, Mandich P, Schenone A, Gambardella A, Bono F, Quattrone A, Devoto M, Monaco AP. Charcot-Marie-Tooth type 4B is caused by mutations in the gene encoding myotubularin-related protein-2 - Nat Genet. 2000 May;25(1):17-9).

Myotubularins and myotubularin-related proteins (MTM) define a conserved protein family implicated in different neuromuscular diseases (Raess, M.A., Friant, S., Cowling, B.S., and Laporte, J. (2016). WANTED - Dead or alive: Myo tubularins,a large disease-associated protein family. Adv Biol Regul.). They have been classified in the phosphatase super-family. In human, eight myotubularins share the C(X)5R motif found in tyrosine and dual-specificity phosphatases and display enzymatic activity, while the other 6 myotubularins lack this motif and are named dead-phosphatases. Unexpectedly, it was found that enzymatically active myotubularins do not act on proteins but dephosphorylate phosphoinositides (PPIn), lipids concentrated in specific membrane sub-domains (Blondeau, F., Laporte, J., Bodin, S., Superti-Furga, G., Payrastre, B., and Mandel, J.L. (2000). Myotubularin, a phosphatase deficient in myotubular myopathy, acts on phosphatidylinositol 3-kinase and phosphatidylinositol 3-phosphate pathway. Hum Mol Genet 9, 2223-2229.; Taylor, G.S., Maehama, T., and Dixon, J.E. (2000)). Inaugural article: myotubularin, a protein tyrosine phosphatase mutated in myotubular myopathy, dephosphorylates the lipid second messenger, phosphatidylinositol 3-phosphate. Proc Natl Acad Sci U S A 97, 8910-8915). PPIn are lipid second messengers implicated in a wide range of cellular processes including signaling and intracellular organization (Vicinanza, M., D'Angelo, G., Di Campli, A., and De Matteis, M.A. (2008). Function and dysfunction of the PI system in membrane trafficking. EMBO J 27, 2457-2470.). Myotubularins are PPIn 3-phosphatases that dephosphorylate the phosphatidylinositol 3-phosphate (PtdIns3P) and the phosphatidylinositol 3,5-bisphosphate (PtdIns(3,5)P2), leading to the production of PtdIns5P (Berger, P., Bonneick, S., Willi, S., Wymann, M., and Suter, U. (2002). Inaugural article: myotubularin, a protein tyrosine phosphatase mutated in myotubular myopathy, dephosphorylates the lipid second messenger, phosphatidylinositol 3-phosphate. Proc Natl Acad Sci U S A 97, 8910-8915; Tronchere, H., Laporte, J., Pendaries, C., Chaussade, C., Liaubet, L., Pirola, L., Mandel, J.L., and Payrastre, B. (2004). Production of phosphatidylinositol 5-phosphate by the phosphoinositide 3-phosphatase myotubularin in mammalian cells. (Tronchère H, Laporte J, Pendaries C, Chaussade C, Liaubet L, Pirola L, Mandel JL, Payrastre B. J Biol Chem. 2004 Feb 20;279(8):7304-12. Epub 2003 Dec 1.). PtdIns5P is implicated in transcriptional regulation and growth factor signaling, while PtdIns3P and PtdIns(3,5)P2 regulate membrane trafficking and autophagy. PtdIns3P is produced through the phosphorylation of PtdIns by class II and III PtdIns 3-kinases and PtdIns(3,5)P2 is obtained mainly from the phosphorylation of PtdIns3P by PIKfyve (Jin, N., Lang, M.J., and Weisman, L.S. (2016). Phosphatidylinositol 3,5-bisphosphate: regulation of cellular events in space and time. Biochem Soc Trans 44, 177-184; Schink, K.O., Raiborg, C., and Stenmark, H. (2013). Phosphatidylinositol 3-phosphate, a lipid that regulates membrane dynamics, protein sorting and cell signalling. Bioessays 35, 900-912). They recruit proteins to specific endosomal pools or to endoplasmic reticulum, allowing the maturation and interconversion of endosomes or the formation of autophagic vacuoles, respectively. For example, the FYVE (Fab1-YOTB-Vac1-EEA1) domain of EEA1 binds specifically PtdIns3P concentrated on early endosomes to regulate endosomal fusion and cargo delivery (Schink et al., 2013, supra). Dead myotubularins oligomerize with and regulate the enzymatic activity and/or subcellular localization of active homologs (Berger, P., Berger, I., Schaffitzel, C., Tersar, K., Volkmer, B., and Suter, U. (2006). Multi-level regulation of myotubularin-related protein-2 phosphatase activity by myotubularin-related protein-13/set-binding factor-2. Hum Mol Genet 15, 569-579.; Kim et al., 2003, supra; Nandurkar, H.H., Layton, M., Laporte, J., Selan, C., Corcoran, L., Caldwell, K.K., Mochizuki, Y., Majerus, P.W., and Mitchell, C.A. (2003). Identification of myotubularin as the lipid phosphatase catalytic subunit associated with the 3-phosphatase adapter protein, 3-PAP. Proc Natl Acad Sci USA 100, 8660-8665). In addition to the active or dead phosphatase domain, myotubularins share a PH-GRAM (Pleckstrin Homology, Glucosyltransferase, Rab-like GTPase Activator and Myotubularin) domain that bind to PPIn or proteins, and coiled-coil domain implicated in their oligomerization (Raess et al., 2016, supra).

There are 14 myotubularins in human and one active myotubularin in yeast (Saccharomyces cerevisiae) (Lecompte, O., Poch, O., and Laporte, J. (2008). PtdIns5P regulation through evolution: roles in membrane trafficking? Trends Biochem Sci 33, 453-460.; Raess et al., 2016, supra). The yeast myotubularin (Ymr1p) regulates vacuole protein sorting and fragmentation (Parrish, W.R., Stefan, C.J., and Emr, S.D. (2004). Essential role for the myotubularin-related phosphatase Ymr1p and the synaptojanin-like phosphatases Sjl2p and Sjl3p in regulation of phosphatidylinositol 3-phosphate in yeast. Mol Biol Cell 15, 3567-3579.). Overexpression of human myotubularin in yeast leads to the enlargement of the vacuole as a consequence of its phosphatase activity (Amoasii, L., Bertazzi, D.L., Tronchere, H., Hnia, K., Chicanne, G., Rinaldi, B., Cowling, B.S., Ferry, A., Klaholz, B., Payrastre, B., et al. (2012). Phosphatase-dead myotubularin ameliorates X-linked centronuclear myopathy phenotypes in mice. PLoS Genet 8, e1002965; Blondeau et al., 2000, supra). As stated previously, in humans, loss-of-function mutations in myotubularin 1 (MTM1) cause the severe congenital myopathy called XLCNM, while mutations in either the active myotubularin-related 2 gene protein (MTMR2) or the dead myotubularin-related protein MTMR13 cause Charcot-Marie-Tooth (CMT) peripheral neuropathies (CMT4B1, OMIM 601382 and CMT4B2, OMIM 604563 respectively) (Azzedine, H., Bolino, A., Taieb, T., Birouk, N., Di Duca, M., Bouhouche, A., Benamou, S., Mrabet, A., Hammadouche, T., Chkili, T., et al. (2003). Mutations in MTMR13, a New Pseudophosphatase Homologue of MTMR2 and Sbf1, in Two Families with an Autosomal Recessive Demyelinating Form of Charcot-Marie-Tooth Disease Associated with Early-Onset Glaucoma. Am J Hum Genet 72, 1141-1153.; Bolino, A., Muglia, M., Conforti, F.L., LeGuern, E., Salih, M.A., Georgiou, D.M., Christodoulou, K., Hausmanowa-Petrusewicz, I., Mandich, P., Schenone, A., et al. (2000). Charcot-Marie-Tooth type 4B is caused by mutations in the gene encoding myotubularin-related protein-2. Nat Genet 25, 17-19; Senderek, J., Bergmann, C., Weber, S., Ketelsen, U.P., Schorle, H., Rudnik-Schonebom, S., Buttner, R., Buchheim, E., and Zerres, K. (2003). Mutation of the SBF2 gene, encoding a novel member of the myotubularin family, in Charcot-Marie-Tooth neuropathy type 4B2/11p15. Hum Mol Genet 12, 349-356). In addition, putative mutations in MTMR5 (Sbf1) were linked to CMT4B3 (OMIM 615284) and axonal neuropathy (Alazami, A.M., Alzahrani, F., Bohlega, S., and Alkuraya, F.S. (2014). SET binding factor 1 (SBF1) mutation causes Charcot-Marie-tooth disease type 4B3. Neurology 82, 1665-1666 ; Manole, A., Horga, A., Gamez, J., Raguer, N., Salvado, M., San Millan, B., Navarro, C., Pittmann, A., Reilly, M.M., and Houlden, H. (2016). SBF1 mutations associated with autosomal recessive axonal neuropathy with cranial nerve involvement. Neurogenetics; Nakhro et al., 2013).

Thus, lack of one myotubularin is not fully compensated by its homologs, while they are ubiquitously expressed. Moreover, the related diseases affect different tissues. Of note, MTM1 and MTMR2 are part of the same evolutionary sub-group based on their sequence (Lecompte et al., 2008, supra). Thus, this suggests uncharacterized tissue-specific functions potentially reflecting different activities or different interactors.

Consequently, there is a significant need for an appropriate centronuclear myopathy treatment, in particular for new and more effective therapeutic agents.

Here, *in vivo* functions of MTM1 and MTMR2 were compared in yeast and mice and it was found that a specific isoform of MTMR2 had the capacity to compensate for the loss of MTM1 quite efficiently. Such MTMR2 form can rescue the myopathy displayed by Mtm1KO mice, which makes it an effective agent for the treatment of centronuclear myopathies and more specifically for the treatment of XLCNM.

### SUMMARY OF THE INVENTION

The present disclosure provides methods and compositions for treating centronuclear myopathies or for treating diseases or disorders associated with MTM1 mutation or deficiency. The present invention provides compositions and methods for treatment of myopathy or diseases or disorders associated with MTM1 mutation or deficiency, in a subject in need thereof. The present invention relates to a method of delivering a specific MTMR2 polypeptide, called herein short isoform of MTMR2, to subjects in need of improved muscle function. The compositions and methods of the present invention increase the formation of muscle and improve muscle function in the subject.

The present invention is useful for treating an individual with XLCNM. The present invention improves muscle function and prolongs survival in afflicted subjects.

In a particular aspect, the present invention concerns a composition comprising a particular MTMR2 polypeptide, called herein short isoform of MTMR2 or a nucleic acid sequence producing or encoding said particular MTMR2 polypeptide. Said composition can be for use in the treatment of centronuclear myopathies or for a treatment of diseases or disorders associated with MTM 1 mutation or deficiency.

The centronuclear myopathy is X-linked CNM (XLCNM).

The present invention also provides isolated polypeptides comprising a short isoform of MTMR2 polypeptide, as well as pharmaceutical compositions comprising a short isoform of MTMR2 polypeptide in combination with a pharmaceutical carrier.

Also disclosed are constructs useful for producing such polypeptide. Further, the present invention relates to methods of making such polypeptides or constructs that encode them. Additionally, disclosed herein are methods of using the said polypeptide, for example, for a treatment of diseases or disorders associated with MTM1 mutation or deficiency.

These and other objects and embodiments of the invention will become more apparent after the detailed description of the invention.

### BRIEF DESCRIPTION OF THE DRAWINGS

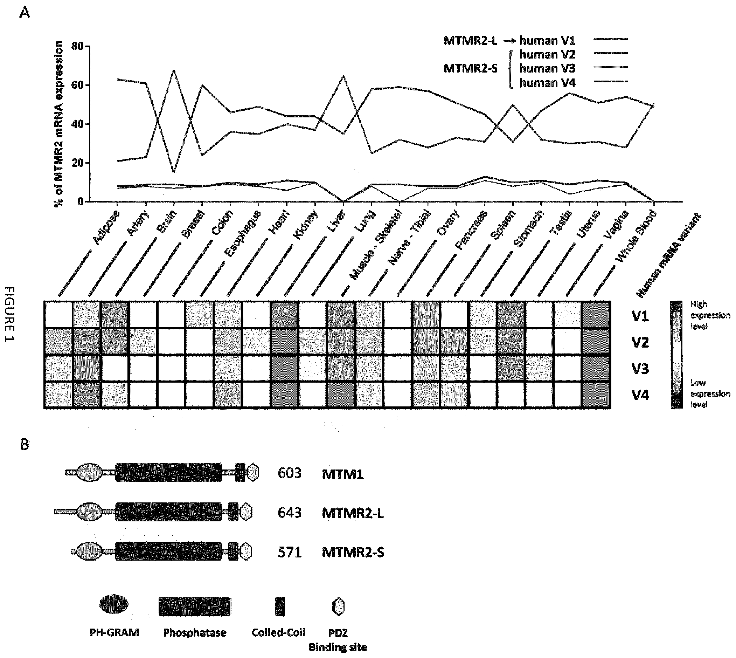

**Figure 1****:** MTMR2 splicing isoforms are differentially expressed and encode for long and short protein isoforms. (**A**) Comparative expression of MTMR2 mRNA isoforms V1 to V4 in 20 human tissues from GTEx database mining (top). Human MTMR2 V2 isoform contains additional exons 1a and 2a compared to V1, V3 contains exon 1a and V4 contains exons 1a and 2b. Tissue expression of each isoform independently (bottom). (**B**) Protein domains MTMR2-L encoded by V1 mRNA isoform, and MTMR2-S encoded by the other isoforms, compared to MTM1.

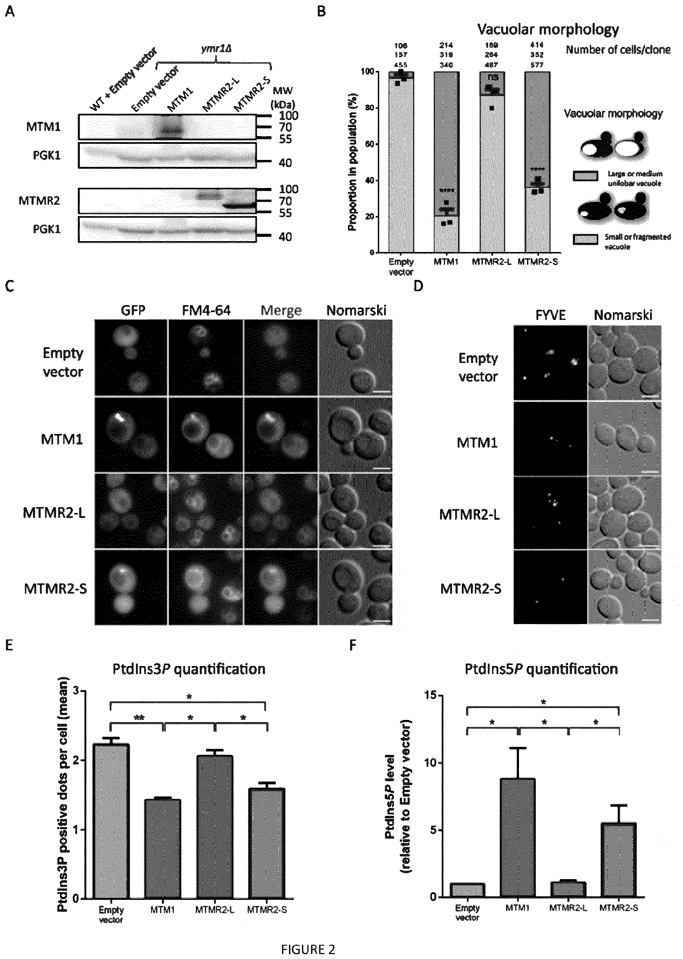

**Figure 2****:** Short but not long MTMR2 isoform displays an MTM1-like activity. Exogenous expression of human MTM1 and MTMR2 long and short isoforms using the high copy number plasmid 2µ in *ymr1Δ* yeast cells. (**A**) Detection of exogenously expressed human myotubularins by western blot using anti-MTM1 or anti-MTMR2 antibodies, in two independents blots with the same samples. Wild-type (WT) and *ymr1Δ* yeast strains with empty vectors are used as controls. Pgk1p is used as a loading control. This blot is representative of at least 3 independent experiments. (**B**) Quantification of vacuolar morphology in yeast cells over-expressing untagged myotubularins. Three clones analyzed per constructs; the number of cells counted per clone is indicated above. Data represent means ± s.e.m. ^{∗∗∗∗}*p*<0.0001, ns not significant (ANOVA test). (**C**) Localization of GFP-tagged human myotubularins. Vacuole morphology is assessed by the lipophilic dye FM4-64 and Nomarski differential contrast. *ymr1Δ* yeast cells and MTMR2-L expressing cells display a fragmented vacuole while MTM1 and MTMR2-S over-expressing cells have a large vacuole. **(D)** FYVE punctuated localization in yeast clones expressing untagged myotubularins and DsRED-tagged FYVE domain that specifically binds PtdIns3*P*. **(E)** PtdIns3*P* quantification by counting the number of FYVE-positive dots per cell, as represented in (D).. PtdIns3*P* is decreased upon MTM1 and MTMR2-S expression but not with MTMR2-L. Data represent means ± s.e.m. ^{∗}*p*<0.05, ^{∗∗}*p*<0.01 (ANOVA test). **(F)** PtdIns5*P* quantification by mass assay on total lipid extract from yeast cells over-expressing untagged myotubularins. Three clones analyzed per constructs. Data represent means ± s.e.m. *p<0.05 (ANOVA test).

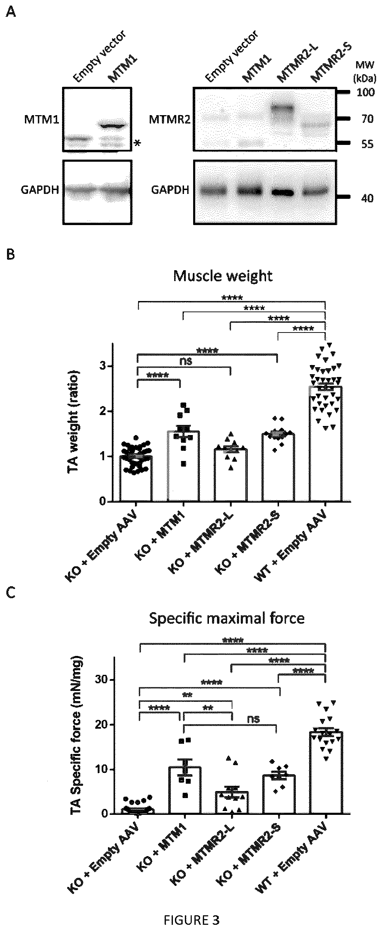

**Figure 3****:** The MTMR2 short isoform rescues muscle weight and force similarly as MTM1 in the *Mtml* KO myopathic mouse. TA muscles from 2-3 week-old *Mtml* KO mice were injected with AAV2/1 expressing myotubularins and analyzed 4 weeks later. **(A)** Detection of exogenously expressed human myotubularins by western blot using anti-MTM1 or anti-MTMR2 antibodies; GAPDH is used as a loading control. Unspecific bands are indicated by a star. This blot is representative for each construct, and at least 10 muscles per construct were analyzed. **(B)** Ratio of muscle weight of TA expressing human myotubularins compared to the contralateral leg injected with empty AAV. MTMR2-S improved muscle mass similarly as MTM1 while MTMR2-L had no effect. A value of 1 was set for the *Mtml* KO mice injected with empty AAV. n>10. Data represent means ± s.e.m. ^{∗∗∗∗}*p*<0.0001, ns not significant (ANOVA test). **(C)** Specific maximal force of TA muscle (absolute values). Both MTMR2 isoforms improved muscle force. n>7. Data represent means ± s.e.m. ^{∗∗}*p*<0.01, ^{∗∗∗∗}*p*<0.0001, ns not significant (ANOVA test).

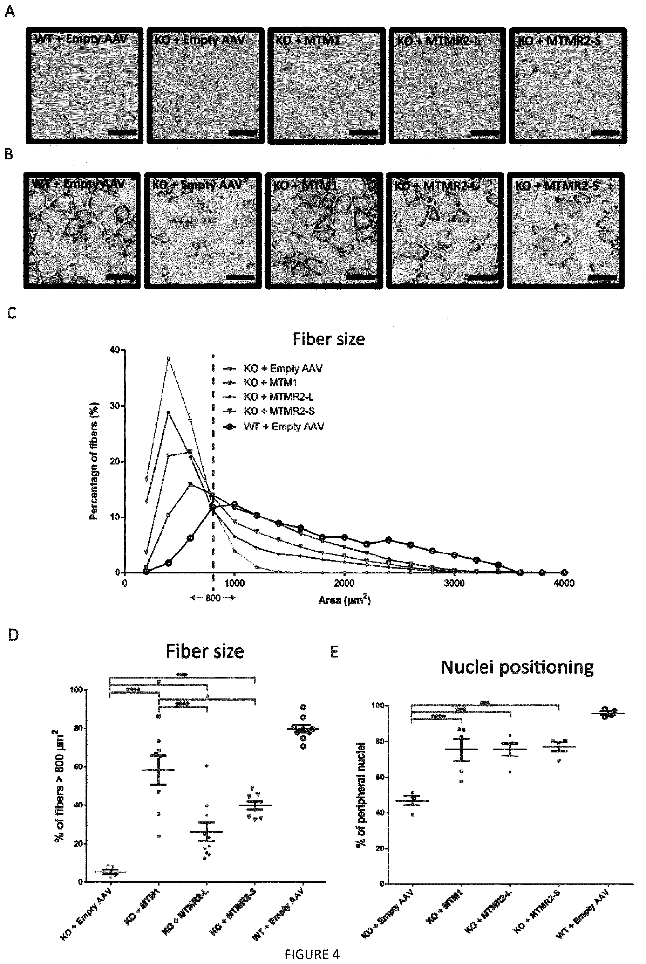

**Figure 4****:** Both long and short MTMR2 isoforms improve the histological hallmarks of the *Mtml* KO mouse. TA muscles from *Mtml* KO mice were injected with AAV2/1 expressing myotubularins 2-3 week-old and analyzed 4 weeks later. **(A)** Hematoxylin-eosin staining of TA muscle sections. Scale bar 100 µm. **(B)** Succinate dehydrogenase (SDH) staining of TA muscle sections. Scale bar 100 µm. **(C)** Quantification of fiber area. Fiber size is grouped into 200 µm² intervals and represented as a percentage of total fibers in each group. n>1000 for 8 mice. **(D)** Percentage of fibers above 800 µm². n>8. Data represent means ± s.e.m. ^{∗}*p*<0.05, ^{∗∗∗}*p*<0.001, ^{∗∗∗∗}*p*<0.0001 (ANOVA test). The value for WT is statistically different from all *Mtml* KO injected groups. **(E)** Nuclei positioning in TA muscle. Percentage of well-positioned peripheral nuclei. n>6 animals. Data represent means ± s.e.m. ^{∗∗∗}*p*<0.001, ^{∗∗∗∗}*p*<0.0001 (ANOVA test). The value for WT is statistically different from all *Mtml* KO injected groups.

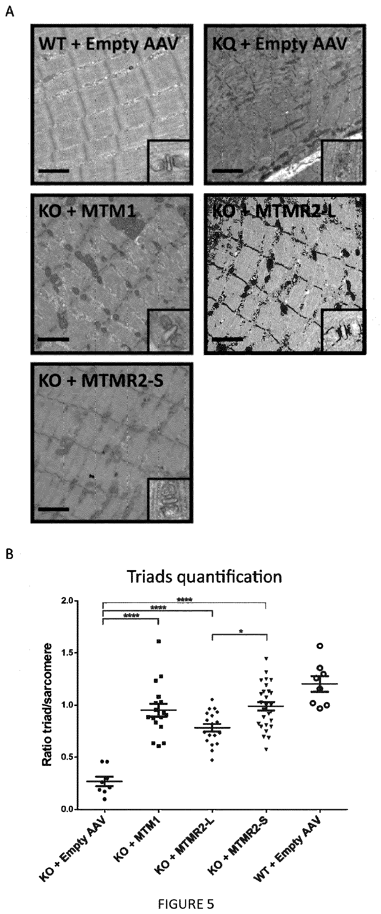

**Figure 5****:** MTMR2 isoforms rescue the muscle ultrastructure and triad morphology of the *Mtml* KO muscles. TA muscles from *Mtml* KO mice were injected with AAV2/1 expressing myotubularins. **(A)** Electron microscopy pictures displaying sarcomere, mitochondria and triad organization. Scale bar 1 µm. Representative triads are displayed in the zoom square. **(B)** Quantification of the number of well-organized triads per sarcomere. n>20 images for 2 mice each. All muscles expressing myotubularins quantify differently than the *Mtml* KO. Data represent means ± s.e.m. ^{∗}*p*<0.05, ^{∗∗∗∗}*p*<0.0001 (ANOVA test).

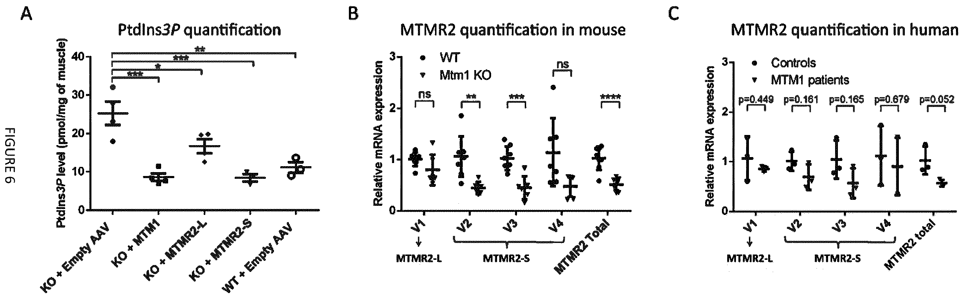

**Figure 6****:** The MTMR2-S short isoform is reduced in the *Mtml* KO mouse and its overexpression normalizes PtdIns3*P* level. (A) Quantification of PtdIns3*P* level by competitive ELISA in TA muscles from *Mtml* KO mice expressing different myotubularins and in WT muscles. n>3 mice. Data represent means ± s.e.m. ^{∗}*p*<0.05, ^{∗∗}*p*<0.01, ^{∗∗∗}*p*<0.001 (ANOVA test). PtdIns3*P* levels in *Mtml* KO muscles expressing the different myotubularins are not statistically different from the WT controls. **(B)** Quantification by qRT-PCR of MTMR2 isoforms (V1 to V4) in the TA muscle *of Mtml* KO mice compared to WT mice. n>6. Each isoform is presented as an independent ratio, with a value of 1 set for expression in WT mice. Data represent means ± s.d. ^{∗∗}*p*<0.01, ^{∗∗∗}*p*<0.001, ^{∗∗∗∗}*p*<0.0001, ns not significant (Student's t-test). **(C)** Quantification by qRT-PCR of MTMR2 isoforms (V1 to V4) in muscles of MTM1 patients compared to controls. N=3. Each isoform is presented as an independent ratio, with a value of 1 set for expression in control patients. Data represent means ± s.d. The *P* value is indicated for each isoform (Student's t-test).

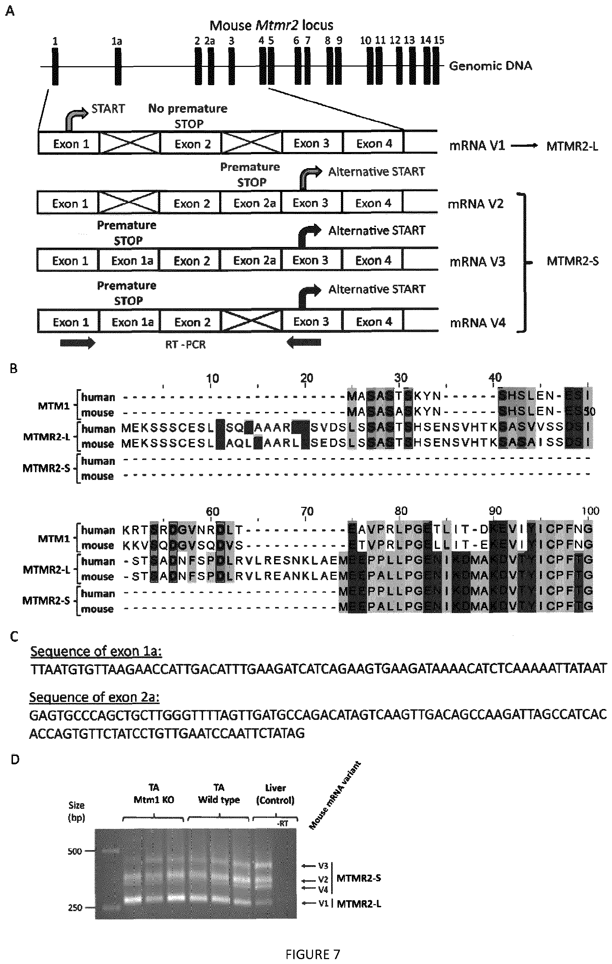

**Figure 7****:** MTMR2 mRNA and protein isoforms in human and mouse. **(A)** Genomic structure and mRNA isoforms of MTMR2 in mouse. Inclusion of any combination of the alternative exons 1a or 2a brings a premature stop codon and unmasks an alternative start site in exon 3. Murine MTMR2 V1 encodes for the MTMR2-L while isoforms V2 to V4 encode for MTMR2-S. **(B)** Protein alignment of the N-terminal region of human and mouse MTM1, MTMR2-L and MTMR2-S. The PH-GRAM domain starts at position 75. **(C)** Sequence of mouse alternative exons 1a and 2a from sequencing of RT-PCR products from muscle. **(D)** PCR between exons 1 and 3 of MTMR2 on cDNA from TA muscles isolated from WT and *Mtml* KO mice and from WT liver. The 4 mRNA variants are detected.

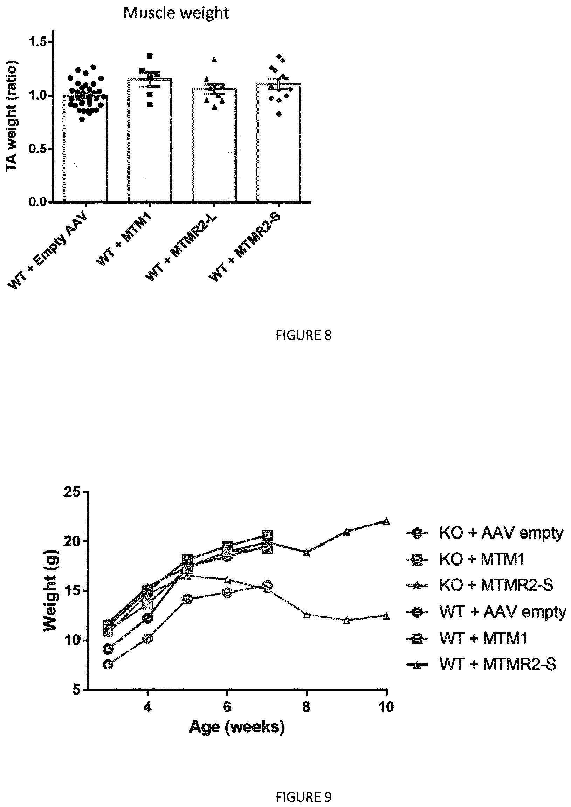

**Figure 8****:** Expression of MTMR2 isoforms does not induce muscle hypertrophy in WT mice. TA muscles from WT mice were injected with AAV2/1 expressing myotubularins at 3 week-old and analyzed 4 weeks later. Ratio of muscle weight of TA expressing human myotubularins compared to the contralateral leg injected with empty AAV. A value of 1 is set for the WT TA muscle weight. n>5. Data represent means ± s.e.m. No significant differences (ANOVA test).
**Figure 9****:** MTMR2-S isoform improves the body weight of myopathic mice. Measure of the body weight from 3 weeks to maximum 10 weeks of age of Mtm1 KO or WT mice overexpressing the different myotubularins.

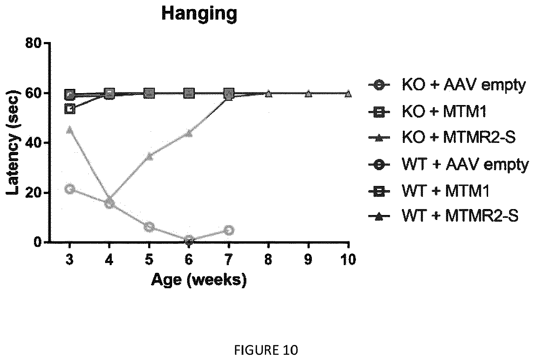

**Figure 10****:** MTMR2-S isoform rescue the muscle force of *Mtml* KO mice. The muscle strength of *Mtml* KO or WT mice overexpressing the different myotubularins was assessed by hanging test each week from 3 to 10 weeks of age.

### DETAILED DESCRIPTION

Unless otherwise defined, all technical and scientific terms used herein have the same meaning as commonly understood by one of ordinary skill in the art to which this invention belongs.

The articles "a" and "an" are used herein to refer to one or to more than one (i.e., to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1 % from the specified value, as such variations are appropriate to perform the disclosed methods or compositions.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

According to the invention, the term "comprise(s)" or "comprising" (and other comparable terms, e.g., "containing," and "including") is "open-ended" and can be generally interpreted such that all of the specifically mentioned features and any optional, additional and unspecified features are included. According to specific embodiments, it can also be interpreted as the phrase "consisting essentially of' where the specified features and any optional, additional and unspecified features that do not materially affect the basic and novel characteristic(s) of the claimed invention are included or the phrase "consisting of" where only the specified features are included, unless otherwise stated.

The terms "polypeptide," "peptide" and "protein" are used interchangeably herein to refer to a polymer of amino acid residues covalently linked by peptide bonds. The terms apply to amino acid polymers in which one or more amino acid residue is an artificial chemical mimetic of a corresponding naturally occurring amino acid, as well as to naturally occurring amino acid polymers and non-naturally occurring amino acid polymers. "Polypeptides" include, for example, biologically active fragments, substantially homologous polypeptides, oligopeptides, homodimers, heterodimers, variants of polypeptides, modified polypeptides, derivatives, analogues, fusion proteins, among others. The polypeptides include natural peptides, recombinant peptides, synthetic peptides, or a combination thereof.

As used herein, "treating a disease or disorder" means reducing the frequency with which a symptom of the disease or disorder is experienced by a patient. Disease and disorder are used interchangeably herein. To "treat" a disease as the term is used herein, means to reduce the frequency or severity of at least one sign or symptom of a disease or disorder experienced by a subject. Within the context of the invention, the term treatment denotes curative, symptomatic, and preventive treatment. As used herein, the term "treatment" of a disease refers to any act intended to extend life span of subjects (or patients) such as therapy and retardation of the disease progression. The treatment can be designed to eradicate the disease, to stop the progression of the disease, and/or to promote the regression of the disease. The term "treatment" of a disease also refers to any act intended to decrease the symptoms associated with the disease, such as hypotonia and muscle weakness. More specifically, the treatment according to the invention is intended to delay the appearance of the centronuclear myopathy phenotypes or symptoms, ameliorate the motor and/or muscular behavior and/or lifespan.

A disease or disorder is "alleviated" if the severity of a symptom of the disease or disorder, the frequency with which such a symptom is experienced by a patient, or both, is reduced. A "therapeutic" treatment is a treatment administered to a subject who exhibits signs of pathology, for the purpose of diminishing or eliminating at least one or all of those signs.

The phrase "therapeutically effective amount," as used herein, refers to an amount that is sufficient or effective to prevent or treat (delay or prevent the onset of, prevent the progression of, inhibit, decrease or reverse) a disease or disorder, including provision of a beneficial effect to the subject or alleviating symptoms of such diseases.

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human. Preferably the subject is a human patient whatever its age or sex. New-borns, infants, children are included as well.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

"Expression vector" refers to a vector comprising a recombinant polynucleotide comprising expression control sequences operatively linked to a nucleotide sequence to be expressed, which is referred herein as a construct. An expression vector comprises sufficient cis-acting elements for expression; other elements for expression can be supplied by the host cell or in an in vitro expression system. Expression vectors include all those known in the art, such as cosmids, plasmids (e.g., naked or contained in liposomes) and viruses (e.g., lentiviruses, retroviruses, adenoviruses, and adeno-associated viruses) that incorporate the recombinant polynucleotide. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like. The construct is therefore incorporated into an expression vector.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared×100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain (an) intron(s).

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR^{™}, and the like, and by synthetic means.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

The MTMR2 polypeptide of the present invention (called herein short isoform MTMR2 or MTMR2-S) is preferably a short spliced naturally occurring isoform of the human MTMR2 which is of 571 amino acids length. Said MTMR2-S polypeptide is represented by SEQ ID NO: 1. More specifically, said short isoform of MTMR2 polypeptide does not comprise the naturally occurring long chain human MTMR2 polypeptide.

It is disclosed herein that said isoform of MTMR2 represented by SEQ ID NO: 1 has the capacity to compensate for the loss of MTM1 quite efficiently. Such MTMR2-S isoform can rescue the myopathy displayed by Mtm1KO mice, which makes it an effective agent for the treatment of centronuclear myopathies and more specifically for the treatment of XLCNM. This method can lead to sustained improvements in muscle strength, size, and function.

In one aspect, the MTMR2-S used herein comprises an amino acid sequence at least 90% identical (or homologous) to SEQ ID NO: 1 or a bioactive fragment or variant thereof. In some embodiments, the MTMR2 polypeptide comprises an amino acid sequence at least 80%, 85%, 90%, 95%, 97%, 98%, 99% or 100% identical to SEQ ID NO: 1 and is or less than 571 amino acids length, or a bioactive fragment or variant thereof.

As used herein, the MTMR2-S used herein includes various splicing isoforms, fragments, variants, fusion proteins, and modified forms of the short spliced naturally occurring isoform of the human MTMR2 which is of 571 amino acids length, as described above and represented by SEQ ID NO. 1. Such isoforms, fragments or variants, fusion proteins, and modified forms of the naturally occurring isoform MTMR2-S polypeptide have at least a portion of the amino acid sequence of substantial sequence identity to the naturally occurring isoform MTMR2-S polypeptide, and retain at least one function of the naturally occurring MTMR2-S polypeptide. In certain embodiments, a bioactive fragment, variant, or fusion protein of the naturally occurring isoform MTMR2-S polypeptide comprises an amino acid sequence that is at least 80%, 85%, and preferably at least 90%, 95%, 97%, 98%, 99% or 100% identical to the naturally occurring isoform MTMR2-S of SEQ ID No1. As used herein, "fragments" are understood to include bioactive fragments or bioactive variants that exhibit "bioactivity" as described herein. That is, bioactive fragments or variants of MTMR2-S exhibit bioactivity that can be measured and tested. For example, bioactive fragments or variants exhibit the same or substantially the same bioactivity as native (i.e., wild-type, or normal) MTM1 protein, and such bioactivity can be assessed by the ability of the fragment or variant to, e.g., cleave or hydrolyze an endogenous phosphoinositide substrate known in the art, or an artificial phosphoinositide substrate for *in vitro* assays (i.e., a phosphoinositide phosphatase activity). Methods in which to assess any of these criteria are described herein and one must refer more specifically to the examples below where PtdIns3P quantification by ELISA in muscle extracts of Mtm1KO mice expressing the AAV vector or AAV myotubularin constructs were performed, or through the detection of PtdIns3P by a biosensor composed of tandem FYVE protein domain having specific PtdIns3P binding capacities. As stated below in the portions of the examples (see also FIG. 6A), PtdIns3P level was normalized to WT level when expressing MTM1 or the naturally occurring isoform MTMR2-S. As used herein, "substantially the same" refers to any parameter (e.g., activity) that is at least 70% of a control (e.g. KO + MTM1 or WT + empty AAV in the examples) against which the parameter is measured. In certain embodiments, "substantially the same" also refers to any parameter (e.g., activity) that is at least 75%, 80%, 85%, 90%, 92%, 95%, 97%, 98%, 99%, 100%, 102%, 105%, or 110% of a control against which the parameter is measured.

In certain embodiments, any of the foregoing or following MTMR2-S polypeptides disclosed herein are possibly for use in a chimeric polypeptide further comprising one or more polypeptide portions that enhance one or more of *in vivo* stability, *in vivo* half-life, uptake/administration, and/or purification.

The present invention provides a composition that increases the expression of MTMR2-S polypeptide, or a bioactive fragment or variant thereof, in a muscle. For example, in one embodiment, the composition comprises an isolated nucleic acid sequence producing or encoding MTMR2-S polypeptide, or a biologically functional fragment or variant thereof. As described herein, delivery of a composition comprising such nucleic acid sequence improves muscle function. Furthermore, the delivery of a composition comprising such nucleic acid sequence prolongs survival of a subject having a loss of function mutation in MTM1.

The present invention also concerns a pharmaceutical composition comprising a MTMR2-S polypeptide as defined above, or constructs useful for producing such polypeptide, in combination with a pharmaceutical carrier. Also disclosed said compositions for use in the treatment of a centronuclear myopathy or for use in the treatment to improving muscle function.

The present invention further concerns a method for the treatment of a centronuclear myopathy or for the treatment to improving muscle function, wherein the method comprises a step of administering into a subject in need of such treatment a therapeutically efficient amount of the MTMR2-S polypeptide as defined above, or constructs providing the same.

Finally, the present invention concerns the use of the MTMR2-S polypeptide as defined above, or constructs providing the same, for the preparation of a pharmaceutical composition for the treatment of a disease or disorder associated with MTM1 mutation or deficiency, for the treatment of a centronuclear myopathy or for the treatment to improving muscle function.

In one embodiment, the composition comprises an isolated nucleic acid comprising a sequence encoding the MTMR2-S polypeptide or a biologically functional fragment or variant thereof as defined above. In one embodiment, the nucleic acid comprises a sequence comprising at least one of SEQ ID NO: 2. In other embodiments, the nucleic acid comprises a mRNA sequence encoding the MTMR2-S polypeptide or a biologically functional fragment or variant thereof as defined above. In specific embodiments, the nucleic acid comprises a mRNA sequence comprising at least one of SEQ ID NOs: 3, 4 or 5, which are 3 isoforms RNA encoding for the MTMR2-S protein. As stated earlier, the nucleic acid encodes the said short isoform of MTMR2 polypeptide as defined, but does not encode the naturally occurring human MTMR2 polypeptide. The isolated nucleic acid sequence encoding the MTMR2-S polypeptide or a biologically functional fragment or variant thereof as defined above can be obtained using any of the many recombinant methods known in the art, such as, for example by screening libraries from cells expressing the MTMR2 gene, by deriving the gene from a vector known to include the same, or by isolating directly from cells and tissues containing the same, using standard techniques (such as PCR). Alternatively, the gene of interest can be produced synthetically, rather than cloned.

The present invention also includes a vector in which the isolated nucleic acid of the present invention is inserted. The art is replete with suitable vectors that are useful in the present invention. It also refers to a nucleic acid construct or a recombinant host cell comprising a nucleic acid sequence encoding the MTMR2-S polypeptide as defined above; operably linked to one or more control sequences that direct the production of the said polypeptide.

In summary, the expression of natural or synthetic nucleic acids encoding MTMR2-S is typically achieved by operably linking a nucleic acid encoding the MTMR2-S or portions thereof to a promoter, and incorporating the construct into an expression vector. The vectors to be used are suitable for replication and, optionally, integration in eukaryotic cells. Typical vectors contain transcription and translation terminators, initiation sequences, and promoters useful for regulation of the expression of the desired nucleic acid sequence.

The vectors of the present invention may also be used for gene therapy, using standard gene delivery protocols. Methods for gene delivery are known in the art. See, e.g., U.S. Patents Nos. 5,399,346; 5,580,859; or 5,589,466. In another embodiment, the invention provides a gene therapy vector.

The isolated nucleic acid of the invention can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the vector may be provided to a cell in the form of a viral vector. Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Patent No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

For example, vectors derived from retroviruses such as the lentivirus are suitable tools to achieve long-term gene transfer since they allow long-term, stable integration of a transgene and its propagation in daughter cells. In a preferred embodiment, the composition includes a vector derived from an adeno-associated virus (AAV). Adeno-associated viral (AAV) vectors have become powerful gene delivery tools for the treatment of various disorders. AAV vectors possess a number of features that render them ideally suited for gene therapy, including a lack of pathogenicity, minimal immunogenicity, and the ability to transduce postmitotic cells in a stable and efficient manner. Expression of a particular gene contained within an AAV vector can be specifically targeted to one or more types of cells by choosing the appropriate combination of AAV serotype, promoter, and delivery method.

In one embodiment, the MTMR2-S encoding sequence is contained within an AAV vector. More than 30 naturally occurring serotypes of AAV are available. Many natural variants in the AAV capsid exist, allowing identification and use of an AAV with properties specifically suited for skeletal muscle. AAV viruses may be engineered using conventional molecular biology techniques, making it possible to optimize these particles for cell specific delivery of myotubularin nucleic acid sequences, for minimizing immunogenicity, for tuning stability and particle lifetime, for efficient degradation, for accurate delivery to the nucleus, etc.

Among the serotypes of AAVs isolated from human or non-human primates (NHP) and well characterized, human serotype 2 is the first AAV that was developed as a gene transfer vector; it has been widely used for efficient gene transfer experiments in different target tissues and animal models. Clinical trials of the experimental application of AAV2 based vectors to some human disease models are in progress. Other useful AAV serotypes include AAV1, AAV3, AAV4, AAV5, AAV6, AAV7, AAV8, AAV9 and AAV10.

In one embodiment, the vectors useful in the compositions and methods described herein contain, at a minimum, sequences encoding a selected AAV serotype capsid, e.g., an AAV8 capsid, or a fragment thereof. In another embodiment, useful vectors contain, at a minimum, sequences encoding a selected AAV serotype rep protein, e.g., AAV8 rep protein, or a fragment thereof. Optionally, such vectors may contain both AAV cap and rep proteins.

The AAV vectors of the invention further contain a minigene comprising a MTMR2-S nucleic acid sequence producing MTMR2-S polypeptide as described above which is flanked by AAV 5' (inverted terminal repeat) ITR and AAV 3' ITR. A suitable recombinant adeno-associated virus (AAV) is generated by culturing a host cell which contains a nucleic acid sequence encoding an adeno-associated virus (AAV) serotype capsid protein, or fragment thereof, as defined herein; a functional rep gene; a minigene composed of, at a minimum, AAV inverted terminal repeats (ITRs) and a MTMR2-S nucleic acid sequence, or biologically functional fragment thereof; and sufficient helper functions to permit packaging of the minigene into the AAV capsid protein. The components required to be cultured in the host cell to package an AAV minigene in an AAV capsid may be provided to the host cell in trans. Alternatively, any one or more of the required components (e.g., minigene, rep sequences, cap sequences, and/or helper functions) may be provided by a stable host cell which has been engineered to contain one or more of the required components using methods known to those of skill in the art.

In specific embodiments, such a stable host cell will contain the required component(s) under the control of a constitutive promoter. In other embodiments, the required component(s) may be under the control of an inducible promoter. Examples of suitable inducible and constitutive promoters are provided elsewhere herein, and are well known in the art. In still another alternative, a selected stable host cell may contain selected component(s) under the control of a constitutive promoter and other selected component(s) under the control of one or more inducible promoters. For example, a stable host cell may be generated which is derived from 293 cells (which contain E1 helper functions under the control of a constitutive promoter), but which contains the rep and/or cap proteins under the control of inducible promoters. Still other stable host cells may be generated by one of skill in the art.

The minigene, rep sequences, cap sequences, and helper functions required for producing the rAAV of the invention may be delivered to the packaging host cell in the form of any genetic element which transfers the sequences carried thereon. The selected genetic element may be delivered using any suitable method, including those described herein and any others available in the art. The methods used to construct any embodiment of this invention are known to those with skill in nucleic acid manipulation and include genetic engineering, recombinant engineering, and synthetic techniques. Similarly, methods of generating rAAV virions are well known and the selection of a suitable method is not a limitation on the present invention.

Unless otherwise specified, the AAV ITRs, and other selected AAV components described herein, may be readily selected from among any AAV serotype, including, without limitation, AAV1, AAV2, AAV3, AAV4, AAV5, AAV6, AAV7, AAV8, AAV9 and AAV10 or other known or as yet unknown AAV serotypes. These ITRs or other AAV components may be readily isolated from an AAV serotype using techniques available to those of skill in the art. Such an AAV may be isolated or obtained from academic, commercial, or public sources (e.g., the American Type Culture Collection, Manassas, Va.). Alternatively, the AAV sequences may be obtained through synthetic or other suitable means by reference to published sequences such as are available in the literature or in databases such as, e.g., GenBank, PubMed, or the like.

The minigene is composed of, at a minimum, a MTMR2-S encoding nucleic acid sequence (the transgene) and its regulatory sequences, and 5' and 3' AAV inverted terminal repeats (ITRs). In one embodiment, the ITRs of AAV serotype 2 are used. However, ITRs from other suitable serotypes may be selected. It is this minigene which is packaged into a capsid protein and delivered to a selected host cell. The MTMR2-S encoding nucleic acid coding sequence is operatively linked to regulatory components in a manner which permits transgene transcription, translation, and/or expression in a host cell.

In addition to the major elements identified above for the minigene, the AAV vector generally includes conventional control elements which are operably linked to the transgene in a manner which permits its transcription, translation and/or expression in a cell transfected with the plasmid vector or infected with the virus produced by the invention. As used herein, "operably linked" sequences include both expression control sequences that are contiguous with the gene of interest and expression control sequences that act in trans or at a distance to control the gene of interest. Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (i.e., Kozak consensus sequence); sequences that enhance protein stability; and when desired, sequences that enhance secretion of the encoded product. A great number of expression control sequences, including promoters which are native, constitutive, inducible and/or tissue-specific, are known in the art and may be utilized. Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. Depending on the promoter, it appears that individual elements can function either cooperatively or independently to activate transcription.

In order to assess the expression of MTMR2-S, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In one embodiment, the composition comprises a naked isolated nucleic acid encoding MTMR2-S, or a biologically functional fragment thereof, wherein the isolated nucleic acid is essentially free from transfection-facilitating proteins, viral particles, liposomal formulations and the like. It is well known in the art that the use of naked isolated nucleic acid structures, including for example naked DNA, works well with inducing expression in muscle. As such, the present invention encompasses the use of such compositions for local delivery to the muscle and for systemic administration (Wu et al., 2005, Gene Ther, 12(6): 477-486).

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

For use *in vivo,* the nucleotides of the invention may be stabilized, via chemical modifications, such as phosphate backbone modifications (e.g., phosphorothioate bonds). The nucleotides of the invention may be administered in free (naked) form or by the use of delivery systems that enhance stability and/or targeting, e.g., liposomes, or incorporated into other vehicles, such as hydrogels, cyclodextrins, biodegradable nanocapsules, bioadhesive microspheres, or proteinaceous vectors, or in combination with a cationic peptide. They can also be coupled to a biomimetic cell penetrating peptide. They may also be administered in the form of their precursors or encoding DNAs.

Chemically stabilized versions of the nucleotides also include "Morpholinos" (phosphorodiamidate morpholino oligomers - PMO), 2'-O-Methyl oligomers, AcHN-(RXRRBR)2XB peptide-tagged PMO (R, arginine, X, 6-aminohexanoic acid and B, ^{®}-alanine) (PPMO), tricyclo-DNAs, or small nuclear (sn) RNAs. All these techniques are well known in the art. These versions of nucleotides could also be used for exon skipping to promote expression of endogenous MTMR2-S.

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution.

Regardless of the method used to introduce exogenous nucleic acids into a host cell or otherwise expose a cell to the MTMR2-S of the present invention, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

Genome editing can also be used as a tool according to the invention. Genome editing is a type of genetic engineering in which DNA is inserted, replaced, or removed from a genome using artificially engineered nucleases, or "molecular scissors". The nucleases create specific doublestranded break (DSBs) at desired locations in the genome, and harness the cell's endogenous mechanisms to repair the induced break by natural processes of homologous recombination (HR) and non-homologous end-joining (NHEJ). There are currently four families of engineered nucleases being used: Zinc finger nucleases (ZFNs), Transcription Activator-Like Effector Nucleases (TALENs), the CRISPR/Cas system (more specifically Cas9 system, as described by P. Mali et al., in Nature Methods, vol. 10 No. 10, October 2013), or engineered meganuclease re-engineered homing endonucleases. Said nucleases can be delivered to the cells either as DNAs or mRNAs, such DNAs or mRNAs are engineered to produce the MTMR2-S polypeptide according to the invention.

The nucleotides as defined above used according to the invention can be administered in the form of DNA precursors or molecules coding for them.

The MTMR2-S polypeptide as defined above, including fragments or variants thereof, can be chemically synthesized using techniques known in the art such as conventional solid phase chemistry. The fragments or variants can be produced (by chemical synthesis, for instance) and tested to identify those fragments or variants that can function as well as or substantially similarly to a native MTM1 protein, for example, by testing their ability to cleave or hydrolyze a endogenous phosphoinositide substrate or a synthetic phosphoinositide substrate (i.e., phosphoinositide phosphatase activity), recruit and/or associate with other proteins such as, for example, desmin, PI 3-kinase hVps34 or hVps15 (i.e., proper localization), or treat centronuclear myopathies or treat diseases or disorders associated with MTM1 mutation or deficiency.

In certain embodiments, the present invention contemplates modifying the structure of an MTMR2-S polypeptide for such purposes as enhancing therapeutic or prophylactic efficacy, or stability (e.g., *ex vivo* shelf life and resistance to proteolytic degradation *in vivo*). Such modified MTMR2-S polypeptides have the same or substantially the same bioactivity as naturally-occurring (i.e., native or wild-type) MTMR2-S polypeptide. Modified polypeptides can be produced, for instance, by amino acid substitution, deletion, or addition at one or more positions. For instance, it is reasonable to expect, for example, that an isolated replacement of a leucine with an isoleucine or valine, an aspartate with a glutamate, or a similar replacement of an amino acid with a structurally related amino acid (e.g., conservative mutations) will not have a major effect on the biological activity of the resulting molecule. Conservative replacements are those that take place within a family of amino acids that are related in their side chains.

In a particular embodiment, the therapeutically effective amount to be administered according to the invention is an amount sufficient to alleviate at least one or all of the signs of diseases or disorders associated with MTM1 mutation or alteration, including centronuclear myopathy, or to improve muscle function. The amount of MTMR2-S to be administered can be determined by standard procedure well known by those of ordinary skill in the art. Physiological data of the patient (e.g. age, size, and weight), the routes of administration and the disease to be treated have to be taken into account to determine the appropriate dosage, optionally compared with subjects that do not present centronuclear myopathies. One skilled in the art will recognize that the amount of MTMR2-S polypeptide or of a vector containing or expressing the nucleic acid producing MTMR2-S to be administered will be an amount that is sufficient to treat at least one or all of the signs of diseases or disorders associated with MTM1 mutation, including centronuclear myopathy, or to improve muscle function. Such an amount may vary inter alia depending on such factors as the selected DNMR2-S polypeptide or vector expressing the same, the gender, age, weight, overall physical condition of the patient, etc. and may be determined on a case by case basis. The amount may also vary according to other components of a treatment protocol (e.g. administration of other pharmaceuticals, etc.). Generally, when the therapeutic agent is a nucleic acid, a suitable dose is in the range of from about 1 mg/kg to about 100 mg/kg, and more usually from about 2 mg/kg/day to about 10 mg/kg. If a viral-based delivery of the nucleic acid is chosen, suitable doses will depend on different factors such as the virus that is employed, the route of delivery (intramuscular, intravenous, intra-arterial or other), but may typically range from 10⁻⁹ to 10⁻¹⁵ viral particles/kg. Those of skill in the art will recognize that such parameters are normally worked out during clinical trials. Further, those of skill in the art will recognize that, while disease symptoms may be completely alleviated by the treatments described herein, this need not be the case. Even a partial or intermittent relief of symptoms may be of great benefit to the recipient. In addition, treatment of the patient may be a single event, or the patient is administered with the DNMR2-S or nucleic acid encoding the same on multiple occasions, that may be, depending on the results obtained, several days apart, several weeks apart, or several months apart, or even several years apart.

The pharmaceutical composition of the invention is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

Possible pharmaceutical compositions include those suitable for oral, rectal, intravaginal, mucosal, topical (including transdermal, buccal and sublingual), or parenteral (including subcutaneous (sc), intramuscular (im), intravenous (iv), intra-arterial, intradermal, intrasternal, injection, intraperitoneal or infusion techniques) administration. For these formulations, conventional excipient can be used according to techniques well known by those skilled in the art. In particular, intramuscular or systemic administration, such as intraperitoneal administration, is preferred. In order to provide a localized therapeutic effect, specific muscular or intramuscular administration routes are preferred.

Pharmaceutical compositions according to the invention may be formulated to release the active drug substantially immediately upon administration or at any predetermined time or time period after administration.

The following examples are given for purposes of illustration and not by way of limitation.

### EXAMPLES

### Abbreviations used in the specification:

Aa: amino acids
AAV: adeno-associated virus
CMT: Charcot-Marie-Tooth
CNM: centronuclear myopathy
FYVE: Fab1-YOTB-Vac1-EEA1
HE: hematoxylin-eosin
KO: knockout
MTM: myotubularin
MTMR: myotubularin-related
PH-GRAM: Pleckstrin Homology, Glucosyltransferase, Rab-like GTPase Activator and Myotubularin
PPIn: phosphoinositides
PtdIns3P: phosphatidylinositol 3-phosphate
PtdIns(3,5)P2: phosphatidylinositol 3,5-bisphosphate
TA: tibialis anterior
WT: wild type

### Materials and methods

### Plasmids and constructs

The human *MTM1* (1812 bp, 603 aa) and *MTMR2-L* (1932 bp, 643 aa) ORFs were cloned into the pDONR207 plasmid (Invitrogen, Carlsbad, CA) to generate entry clones (pSF108 and pSF98 respectively). The pDONR207-MTMR2-S (1716 bp, 571 aa, pSF101) has been obtained by site-directed mutagenesis on *MTMR2-L* into the pSF98 vector, to delete the 216 first nucleotides corresponding to the 72 first amino acids. Gateway system (Invitrogen, Carlsbad, CA) was used to clone the different myotubularin constructs into yeast destination expression vectors pAG424GPD-ccdB-EGFP (Alberti, S., Gitler, A.D. and Lindquist, S. (2007) A suite of Gateway cloning vectors for high-throughput genetic analysis in Saccharomyces cerevisiae. Yeast, 24, 913-919) and pVV200 (Van Mullem, V., Wery, M., De Bolle, X. and Vandenhaute, J. (2003) Construction of a set of Saccharomyces cerevisiae vectors designed for recombinational cloning. Yeast, 20, 739-746) obtained from the European Saccharomyces cerevisiae Archive for Functional Analysis EUROSCARF, or into a pAAV-MCS vector (CMV promoter). All constructs were verified by sequencing. The pCS211 DsRED-FYVE plasmid was previously described (Katzmann, D.J., Stefan, C.J., Babst, M. and Emr, S.D. (2003) Vps27 recruits ESCRT machinery to endosomes during MVB sorting. J. Cell Biol., 162, 413-423).

### Antibodies

Primary antibodies used were rabbit polyclonal anti-MTM1 (2827), mouse monoclonal anti-MTMR2 (4G3), mouse monoclonal anti-phosphoglycerate Kinase 1 (PGK1, Invitrogen) and mouse monoclonal anti-glyceraldehyde-3-phosphate dehydrogenase (anti-GAPDH, Chemicon by Merck Millipore, Darmstadt, Germany). Anti-MTM1 and anti-MTMR2 antibodies were made onsite at the antibodies facility of the Institut de Génétique et Biologie Moléculaire et Cellulaire (IGBMC). Anti-MTMR2 antibodies were raised against full length human MTMR2 and validated in this study using transfected COS-7 cells. Secondary antibodies against mouse and rabbit IgG, conjugated with horseradish peroxidase (HRP) were obtained from Jackson ImmunoResearch Laboratories (West Grove, PA).

### In vivo models

The S. cerevisiae *ymr1Δ* (MATα, ura3-52, leu2-3,112, his3-Δ200, trp1-Δ901, lys2-801, suc2-Δ9 ymr1::HIS3) (14) and WT (MATα, his3Δ1, leu2Δ0, lys2Δ0, ura3Δ0) strains were grown at 30°C in rich medium (YPD): 1% yeast extract, 2% peptone, 2% glucose or synthetic drop-out medium (SC): 0.67% yeast nitrogen base without amino acids, 2% glucose and the appropriate amino acids mixture to ensure plasmid maintenance. The *ymr1Δ* (MATα, his3Δ1, leu2Δ0, lys2Δ0, ura3Δ0, ymr1::KanMX) in the BY4742 background from the yeast systematic deletion collection was not used, because it does not have the *ymr1Δ* phenotype described by Scott D Emr's laboratory (Parrish, W.R., Stefan, C.J. and Emr, S.D. (2004) Essential role for the myotubularin-related phosphatase Ymrlp and the synaptojanin-like phosphatases Sjl2p and Sjl3p in regulation of phosphatidylinositol 3-phosphate in yeast. Mol. Biol. Cell, 15, 3567-3579.).

In this study, wild-type and *Mtml* KO 129 PAS mice were used. The *Mtml* KO mice are characterized by a progressive muscle atrophy and weakness starting at 2-3 weeks and leading to death by 8 weeks (30). Animals were housed in a temperature-controlled room (19-22°C) with a 12:12-h light/dark cycle.

### Bioinformatics analyses

Expression levels of MTMR2 mRNA isoforms was obtained by mining the Genotype-Tissue Expression (GTEx, www.gtexportal.org/home/) database, which has been built by systematic RNA-sequencing using samples of 51 different tissues from hundreds of donors and 2 transformed cell types in culture. This data were then used to calculate the relative expression of MTMR2 mRNA isoforms in the 20 most relevant tissues, and to create a heat map underlining in which tissue a specific isoform is the most/least expressed.

Alignment of the N-terminal part of MTM1, MTMR2-L and MTMR2-S was done using Jalview (www.jalview.org/) and aligning amino acids were identified by Clustalx color coding.

### Expression analysis

Total RNA was purified from tibialis anterior (TA) muscle and liver of 7 week-old wild-type and *Mtml* KO mice, or from muscle biopsies of XLCNM patients and controls, using trizol reagent (Invitrogen, Carlsbad, CA) according to the manufacturer's instructions. cDNAs were synthesised from 500 ng of total RNA using Superscript II reverse transcriptase (Invitrogen) and random hexamers.

PCR amplification of 1/10 diluted cDNA from TA muscle and liver was performed using a forward primer from the 5'-UTR of *MTMR2*:
SEQ ID NO 6: 5'-AGCGGCCTCCAGTTTCTCGCGC-3'
and a reverse primer from exon 3:
   SEQ ID NO 7: 5'-TCTCTCCTGGAAGCAGGGCTGGTTCC-3',
   for 35 cycles of amplification at 72°C (and 65°C as melting temperature) and 30 min of final extension at 72°C, as previously described (Bolino, A., Marigo, V., Ferrera, F., Loader, J., Romio, L., Leoni, A., Di Duca, M., Cinti, R., Cecchi, C., Feltri, M.L. et al. (2002) Molecular characterization and expression analysis of Mtmr2, mouse). The products were analyzed on a 2% agarose gel, each band has been purified using Nucleospin Gel and PCR cleanup kit (Macherey-Nagel, Düren, Germany), then cloned into a pJet2.1 vector using the CloneJet PCR cloning kit (ThermoFisher Scientific, Waltham, MA), and sequenced by Sanger.

Quantitative PCR amplification of 1/10 diluted cDNAs from mouse TA muscles or human muscle biopsies was performed on Light-Cycler 480 II instrument (Roche, Basel, Swiss) using 53°C as melting temperature. Specific sets of primers were used for each mouse *MTMR2* isoform:
SEQ ID NO 8: forward 5'-GACTCACTGTCCAGTGCTTC-3' and
SEQ ID NO 9: reverse 5'-CCTCCCTCAGGACCCTCA-3' for mouse V1,
SEQ ID NO 10: forward 5'-GACTCACTGTCCAGTGCTTC-3' and
SEQ ID NO 11: reverse 5'-CAGCTGGGCACTCCCTCA-3' for mouse V2,
SEQ ID NO 12: forward 5'-AAGATAAAACATCTCAAAAATTATAATTGCTTC-3' and
SEQ ID NO 13: reverse 5'-CAGCTGGGCACTCCCTCA-3' for mouse V3,
SEQ ID NO 14: forward 5'-AAGATAAAACATCTCAAAAATTATAATTGCTTC-3' and
SEQ ID NO 15: reverse 5'-GACTCACTGTCCAGTGCTTC-3' for mouse V4.

Another set of primers (SEQ ID NO 16: forward 5'-TCCTGTGTCTAATGGCTTGC-3' and SEQ ID NO 17: reverse 5'-AACCAAGAGGGCAGGATATG-3') amplifying a sequence common to all mouse isoforms has been used to quantify total mouse MTMR2. Other specific sets of primers were used for each human *MTMR2* isoform:
SEQ ID NO 18: forward 5'-ACTCCTTGTCCAGTGCCTC-3' and
SEQ ID NO 19: reverse 5'-GACTCCCTCAGGACCCTC-3' for human V1,
SEQ ID NO 20: forward 5'-AAGATAAAACATCTCAAAAATTATAATTGCCTC-3' and
SEQ ID NO 21: reverse 5'-GACTCCCTCAGGACCCTC-3' for human V2,
SEQ ID NO 22: forward 5'-AAGATAAAACATCTCAAAAATTATAATTGCCTC-3' and
SEQ ID NO 23: reverse 5'-GAGCGAGACTCCCTCCTC-3' for human V3,
SEQ ID NO 24: forward 5'-AAGATAAAACATCTCAAAAATTATAATTGCCTC-3' and
SEQ ID NO 25: reverse 5'-CTGGACTGCATGGGCCTC-3' for human V4.

Another set of primers (SEQ ID NO 26: forward 5'-TTTCCTGTCTCTAATAACCTGCC-3' and SEQ ID NO 27: reverse 5'-CCAGGAGGGCAGGGTATG-3') amplifying a sequence common to all human isoforms has been used to quantify total human MTMR2. For all qPCR, the *HPRT* gene expression was used as control because of the non-variation in its expression between control and XLCNM muscles.

### Western blot

Total proteins were extracted from yeast cells (OD₆₀₀ₙₘ=0.5-0.9, minimum 3 clones per construct) by TCA precipitation and NaOH lysis (45), and from TA muscles (minimum 10 muscles per construct) by homogenization in RIPA buffer using a tissue homogenizer (Omni TH, Kennesaw, GA). Protein lysates were analyzed by SDS-PAGE and Western blotting on nitrocellulose membrane. Proteins were detected using primary antibody (anti-MTM1 1/500, anti-MTMR2 1/1000, anti-PGK1 1/1000 and anti-GAPDH 1/1000) followed by incubation with the secondary antibody coupled to HRP, and extensive washing. Membranes were revealed by ECL chemiluminescent reaction kit (Supersignal west pico kit, ThermoFisher Scientific, Waltham, MA).

### Yeast phenotyping

*ymr1Δ* yeast cells were transformed using the LiAc-PEG method (46) by yeast expression plasmids pAG424GPD-ccdB-EGFP (2µ, GFP tag at C-ter) or pVV200 (2µ, no tag) containing *MTM1, MTMR2-L* or *MTMR2-S* cDNA. Yeast cells transformed by empty plasmids were used as controls.

For vacuole staining, 1 OD₆₀₀ₙₘ unit of cells was harvested by a 500xg centrifugation for 1 min, incubated in 50 µl YPD medium with 2 µl FM4-64 (200 µM, Invitrogen) for 15 min at 30°C, prior washing with 900 µl YPD and chasing by incubation at 30°C for 10 min followed by a second wash in SC complete medium, the stained living yeast cells were observed by fluorescent microscopy. Between 100 and 600 cells per clone (three different clones per construct) were counted and classified into two categories: large or medium unilobar vacuole, and small or fragmented vacuole.

For PtdIns*3P* quantification, yeast cells were co-transformed by a pVV200 plasmid (empty or containing *MTM1, MTMR2-L* or *MTMR2-S* cDNA) and the pCS211 plasmid expressing the DsRED-FYVE reporter for PtdIns3*P*-enriched membrane structures (Katzmann, D.J., Stefan, C.J., Babst, M. and Emr, S.D. (2003) Vps27 recruits ESCRT machinery to endosomes during MVB sorting. J. Cell Biol., 162, 413-423). After fluorescence microscopy, the number of dots per cell was quantified on minimum 100 cells per clone (2 different clones per construct). For PtdIns5*P* quantification, yeast *ymr1Δ* cells producing the different MTM1 and MTMR2 constructs were grown to exponential phase. Lipid extraction was done as described in Hama *et al.* on 200 OD₆₀₀ₙₘ units of cells (Hama, H., Takemoto, J.Y. and DeWald, D.B. (2000) Analysis of phosphoinositides in protein trafficking. Methods, 20, 465-473.). PtdIns5*P* intracellular levels were determined as described in Morris J.B. et al. Quantification of the PtdIns(5)P level was performed as described by Morris et al. (Morris, J.B., Hinchliffe, K.A., Ciruela, A., Letcher, A.J. and Irvine, R.F. (2000) Thrombin stimulation of platelets causes an increase in phosphatidylinositol 5-phosphate revealed by mass assay. FEBS Lett., 475, 57-60.) and the results were normalized based on the total lipid concentration.

All fluorescence microscopy observations were done with 100X/1.45 oil objective (Zeiss) on a fluorescence Axio Observer D1 microscope (Zeiss) using GPF or DsRED filter and DIC optics. Images were captured with a CoolSnap HQ2 photometrix camera (Roper Scientific) and treated by ImageJ (Rasband W.S., ImageJ, U. S. National Institutes of Health, Bethesda, Maryland, USA, http ://imagej .nih.gov/ij/).

### PtdIns3P quantification by ELISA in muscle extracts

PtdIns3P Mass ELISAs were performed on lipid extracts from whole tibialis anterior (TA) muscle preparations according to the manufacturer's recommendations and using the PtdIns3*P* Mass ELISA kit (Echelon Biosciences, Salt Lake City, UT). TA muscles from 7 week-old wild-type of *Mtml* KO mice were weighed, grinded into a powder using a mortar and pestle under liquid nitrogen and then incubated in ice cold 5% TCA to extract lipids. Extracted lipids were resuspended in PBS-T with 3% protein stabilizer and then spotted on PtdIns3*P* Mass ELISA plates in duplicates. PtdIns3*P* levels were detected by measuring absorbance at 450 nm on a plate reader. Specific amounts were determined by comparison of values to a standard curve generated with known amounts of PtdIns3*P*.

### AAV production

rAAV2/1 vectors were generated by a triple transfection of AAV-293 cell line with pAAV2-insert containing the insert under the control of the CMV promoter and flanked by serotype-2 inverted terminal repeats, pXR1 containing *rep* and *cap* genes of AAV serotype-1, and pHelper encoding the adenovirus helper functions. Viral vectors were purified and quantified by real time PCR using a plasmid standard pAAV-eGFP. Titers are expressed as viral genomes per ml (vg/ml) and rAAV titers used here were 5-7.10¹¹ vg/ml.

### AAV transduction of tibialis anterior muscles of wild-type and Mtm1 KO mice

Two- to 3-week-old wild-type or *Mtml* KO male 129PAS mice were anesthetized by intraperitoneal injection of 5 ml/g of ketamine (20 mg/mL; Virbac, Carros, France) and xylazine (0.4%, Rompun; Bayer, Wuppertal, Germany). Tibialis anterior (TA) muscles were injected with 20 ml of AAV2/1 preparations or sterile AAV2/1 empty vector. Four weeks later, mice were anesthetized and the TA muscle was either functionally analyzed (as described below), or directly dissected and frozen in nitrogen-cooled isopentane for histology, or fixed for electron microscopy (as described below).

### AAV transduction of wild-type and Mtm1 KO mice

For systemic injections, wild type or *Mtml* KO pups were intraperitoneally injected at birth or at Day 1 by 1.5×10¹² units of empty AAV viral particles or AAV overexpressing human MTM1 or MTMR2-S. Then 3 weeks after injection the body weight and the mice skeletal muscle strength were analyzed weekly by two different tests: the grip test and the hanging test (described below).

### Functional analysis of the muscle

Muscle force measurements were evaluated by measuring *in situ* muscle contraction in response to nerve and muscle stimulation, as described previously (Cowling, B.S., Chevremont, T., Prokic, I., Kretz, C., Ferry, A., Coirault, C., Koutsopoulos, O., Laugel, V., Romero, N.B. and Laporte, J. (2014) Reducing dynamin 2 expression rescues X-linked centronuclear myopathy. J Clin Invest, 124, 1350-1363). Animals were anesthetized by intraperitoneal injection of pentobarbital sodium (50 mg per kg). The distal tendon of the TA was detached and tied with a silk ligature to an isometric transducer (Harvard Bioscience, Holliston, MA). The sciatic nerve was distally stimulated, response to tetanic stimulation (pulse frequency of 50 to 143 Hz) was recorded, and absolute maximal force was determined. After contractile measurements, the animals were sacrificed by cervical dislocation. To determine specific maximal force, TA muscles were dissected and weighed.

### Histology

**For intramuscular injections,** transverse cryosections (9 µm) of mouse TA skeletal muscles were stained with hematoxylin and eosin (HE) or Succinate dehydrogenase (SDH) and viewed with a NanoZoomer 2.0HT slide scanner (Hamamatsu, Hamamatsu city, Japan). Fiber area was analyzed on HE sections, using the RoiManager plugin of ImageJ image analysis software. The percentage of peripheral nuclei was counted using the cell counter plugin of ImageJ image analysis software. ImageJ plugins were used to correlate the nuclei positioning to the fiber size, and for the color coding of the myofibers depending on the fiber size.

**For systemic injections,** 5 µm sections from paraffin-embedded organs were prepared, fixed and stained by Haematoxylin and Eosin (H&E). Sections were imaged with a NanoZoomer 2.0HT slide scanner (Hamamatsu).

### Electron microscopy

TA muscles of anesthetized mice were fixed with 4% PFA and 2.5% glutaraldehyde in 0.1 M phosphate buffer (pH 7.2) and processed as described (Cowling, B.S., Toussaint, A., Amoasii, L., Koebel, P., Ferry, A., Davignon, L., Nishino, I., Mandel, J.L. and Laporte, J. (2011) Increased expression of wild-type or a centronuclear myopathy mutant of dynamin 2 in skeletal muscle of adult mice leads to structural defects and muscle weakness. Am. J. Pathol., 178, 2224-2235). Ratio of triads/sarcomere was calculated by dividing number of triad structure identified by the total number of sarcomere present on the section (2 mice per genotype, minimum 10 fibers analyzed per mice, minimum 20 triads per fiber).

### Statistical analysis

Data are mean ± s.e.m. or ± SD as noted in the figure legend. Statistical analysis was performed using 1-way ANOVA followed by Tukey's multiple comparisons test for all data except for the expression analysis (Fig. 6B-C) where an unpaired 2-tailed Student's *t* test was performed. A *P* value less than 0.05 was considered significant.

### Results

### MTMR2 splicing variants are differentially expressed and encode for long and short protein isoforms

Mutations in the *MTMR2* gene are responsible for Charcot-Marie-Tooth neuropathy (CMT4B1) whereas mutations in MTM1 lead to X-linked centronuclear myopathy (XLCNM), suggesting that these two ubiquitously expressed myotubularins have distinct functions. Most tissues contain more than a single isoform, thus their localization and extent of expression could help explain their different functions. In order to investigate MTMR2 function, its tissue expression and isoforms were first defined. In mice, four *MTMR2* mRNA isoforms (V1 to V4) have been previously reported in peripheral nerves, potentially coding for 2 protein isoforms (Fig. 7A-B). Variants V2 to V4 differ from variant V1 by the inclusion of alternative exons 1a and/or 2a leading to a premature stop codon and unmasking an alternative start site in exon 3. Variant V1 encodes a 643 amino acids protein that can be named MTMR2-L (long) while the other isoforms code for a 571 aa protein named MTMR2-S (short) that was previously detected in various cell lines (Bolino, A., Marigo, V., Ferrera, F., Loader, J., Romio, L., Leoni, A., Di Duca, M., Cinti, R., Cecchi, C., Feltri, M.L. et al. (2002) Molecular characterization and expression analysis of Mtmr2, mouse homologue of MTMR2, the Myotubularin-related 2 gene, mutated in CMT4B. Gene, 283, 17-26). The two protein isoforms differ only in their translation start sites; MTMR2-S starts right before the PH-GRAM domain while the MTMR2-L has an extended N-terminal sequence without known homology to any protein domain and that was not visible in the crystal structure (Fig. 1C; Fig. 7B) (Begley, M.J., Taylor, G.S., Brock, M.A., Ghosh, P., Woods, V.L. and Dixon, J.E. (2006) Molecular basis for substrate recognition by MTMR2, a myotubularin family phosphoinositide phosphatase. Proc. Natl. Acad. Sci. U. S. A., 103, 927-932. ; Begley, M.J., Taylor, G.S., Kim, S.A., Veine, D.M., Dixon, J.E. and Stuckey, J.A. (2003) Crystal structure of a phosphoinositide phosphatase, MTMR2: insights into myotubular myopathy and Charcot-Marie-Tooth syndrome. Mol. Cell, 12, 1391-1402). The expression level of these isoforms was first investigated in human through mining the GTEx expression database encompassing data on 51 human tissues (GTEx_consortium. (2015) Human genomics. The Genotype-Tissue Expression (GTEx) pilot analysis: multitissue gene regulation in humans. Science, 348, 648-660). Variant V1 is the major *MTMR2* RNA in brain, liver and spleen while variant V2 is predominant in the other tissues. The different variants were only poorly expressed in skeletal muscle (Fig. 1A). In mouse, RT-PCR and Sanger sequencing confirmed the existence of the four *MTMR2* mRNA variants (V1 to V4) in tibialis anterior (TA) skeletal muscle of wild type (WT) and *Mtm1* KO mice and in the liver (Fig. 7C-1D), suggesting that both MTMR2-L and MTMR2-S proteins are present in skeletal muscle.

### Short but not long MTMR2 isoform displays an MTM1-like activity in yeast cells

To compare the cellular function of MTM1, MTMR2-L and MTMR2-S proteins *in vivo,* Heterologous expression of these human myotubularins in yeast cells was used. Yeast is a good model to study phosphoinositide-dependent membrane trafficking as it is conserved from yeast to higher eukaryotes (Katzmann, D.J., Stefan, C.J., Babst, M. and Emr, S.D. (2003) Vps27 recruits ESCRT machinery to endosomes during MVB sorting. J. Cell Biol., 162, 413-423). In yeast cells, vacuole volume, morphology, acidity and membrane potential are controlled by PtdIns(3,5)*P₂* that is produced through the phosphorylation of PtdIns3P by Fab1/PIKfyve kinase. In *fab1Δ* mutant cells, the vacuole is very large and unilobed due to low levels of PtdIns(3,5)*P₂*. On the contrary, *ymr1Δ* cells lacking the unique yeast myotubularin have fragmented vacuoles due to excess of PtdIns(3,5)*P₂* and/or PtdIns3*P* (14), and this phenotype is complemented by the expression of the human MTM1 that induces a large vacuole phenotype (Amoasii, L., Bertazzi, D.L., Tronchere, H., Hnia, K., Chicanne, G., Rinaldi, B., Cowling, B.S., Ferry, A., Klaholz, B., Payrastre, B. et al. (2012) PLoS Genet, 8, e1002965). To determine MTM1, MTMR2-L and MTMR2-S intracellular localization, GFP-tagged fusions was overexpressed in *ymr1Δ* cells. MTM1-GFP and MTMR2-S-GFP proteins were concentrated to a membrane punctate structure adjacent to the vacuole (also positive for the FM4-64 lipid dye), while MTMR2-L-GFP was mainly in the cytoplasm (Fig. 2C). The vacuolar morphology upon overexpression of either GFP-tagged or untagged human myotubularins in *ymr1Δ* cells by staining the vacuolar membrane with the lipophilic dye FM4-64 was assessed (Fig. 2B-C). To detect MTMR2 isoforms, a mouse monoclonal antibody was raised against recombinant full length human MTMR2-L. This antibody was validated on the transformed yeast protein extracts, and specifically recognized MTMR2-L and MTMR2-S (Fig. 2A). Vacuoles were significantly enlarged upon expression of MTM1 or MTMR2-S in *ymr1Δ* cells while they remained fragmented with MTMR2-L. MTM1 and MTMR2-S are inducing a large vacuolar morphology mimicking a *fab1Δ* phenotype due to the high expression levels of these phosphatases (overexpression plasmid). These results show that only the membrane localized myotubularin constructs rescued the vacuole morphology defects of *ymr1Δ* cells. Since the vacuolar morphology reflects the PtdIns(3,5)*P₂* level and as PtdIns(3,5)*P₂* is not abundant enough to be detected in normal growth conditions (Dove, S.K., Cooke, F.T., Douglas, M.R., Sayers, L.G., Parker, P.J. and Michell, R.H. (1997) Osmotic stress activates phosphatidylinositol-3,5-bisphosphate synthesis. Nature, 390, 187-192), it was quantified by mass assay the level of PtdIns5*P*, the lipid produced by myotubularin phosphatase activity from PtdIns(3,5)*P₂* (Fig. 2F). PtdIns5P level was increased by MTM1 and MTMR2-S overexpression in *ymr1Δ* cells, while MTMR2-L had no effect. It was also quantified the PtdIns3P myotubularin substrate level, by counting the punctate structures that were positive for DsRED-FYVE, a reporter for PtdIns3*P*-enriched membranes (Katzmann, D.J., Stefan, C.J., Babst, M. and Emr, S.D. (2003) Vps27 recruits ESCRT machinery to endosomes during MVB sorting. J. Cell Biol., 162, 413-423) (Fig. 2D-E). Overexpression of MTM1 and MTMR2-S significantly reduced PtdIns3P level while MTMR2-L had no effect. However, previous data showed MTMR2-L had a strong phosphatase activity *in vitro* (Berger, P., Bonneick, S., Willi, S., Wymann, M. and Suter, U. (2002) Loss of phosphatase activity in myotubularin-related protein 2 is associated with Charcot-Marie-Tooth disease type 4B1. Hum. Mol. Genet., 11, 1569-1579), suggesting that the cytoplasmic localization of this isoform in yeast cells does not allow PPIn substrate dephosphorylation. In conclusion, only MTMR2-S has a similar phosphatase activity and localization as MTM1 in yeast cells, while MTMR2-L behaves differently.

### Exogenous expression of MTMR2 short isoform in the Mtm1 KO mice rescues muscle weight and force similarly to MTM1 expression.

To assess whether in mammals MTMR2-S is also functionally closer to MTM1 compared to MTMR2-L, MTM1, MTMR2-L and MTMR2-S were overexpressed in the *Mtm1* KO mouse and analyzed different myopathy-like phenotypes. The different myotubularins were expressed from Adeno-associated virus AAV2/1 under the control of the CMV promoter and the recombinant virions were injected into the TA muscles of 2-3 week old *Mtm1* KO mice. The *Mtml* KO mice develop a progressive muscle atrophy and weakness starting at 2-3 weeks and leading to death by 8 weeks, the TA muscle being the most affected muscle detected in this model (Buj-Bello et al, 2002 ; Cowling, B.S., Chevremont, T., Prokic, I., Kretz, C., Ferry, A., Coirault, C., Koutsopoulos, O., Laugel, V., Romero, N.B. and Laporte, J. (2014) J Clin Invest, 124, 1350-1363). It was previously shown that AAV-mediated expression of MTM1 for 4 weeks in the TA muscle, corrects the myopathy phenotype in *Mtml* KO mice (Amoasii, L., Bertazzi, D.L., Tronchere, H., Hnia, K., Chicanne, G., Rinaldi, B., Cowling, B.S., Ferry, A., Klaholz, B., Payrastre, B. et al. (2012) PLoS Genet, 8, e1002965). Therefore to determine the impact of introducing MTMR2-L and MTMR2-S into *Mtml* KO mice, the previously described protocol for AAV injections was followed (Amoasii, L., Bertazzi, D.L., Tronchere, H., Hnia, K., Chicanne, G., Rinaldi, B., Cowling, B.S., Ferry, A., Klaholz, B., Payrastre, B. et al. (2012) PLoS Genet, 8, e1002965), using MTM1 as a positive control for the rescue, and empty AAV2/1 as a disease control in the contralateral muscle. The MTM1, MTMR2-L and MTMR2-S human myotubularins were expressed in injected TA, as revealed from anti-MTM1 and anti-MTMR2 western-blot analyzes (Fig. 3A). Endogenous MTMR2 proteins were not detected in muscle injected with empty AAV, most likely due to the low level of endogenous expression (Fig. 3A).

Four weeks after AAV injection, the TA muscle weight of the *Mtml* KO mice was decreased by 2.5 fold compared to WT mice, both injected with empty AAV control. MTM1 or MTMR2-S expression in *Mtml* KO mice increased muscle mass significantly compared to the empty AAV control (1.5 fold), contrary to MTMR2-L (Fig. 3B). To address a potential hypertrophic effect of human MTM1 or MTMR2 constructs in wild type (WT) mice, TA muscle weight of injected WT mice was quantified (Fig. 8). No muscle mass increased was noted with any myotubularins indicating that the amelioration observed in the *Mtml* KO mice was not due to a hypertrophic effect but to a functional rescue.

The *Mtml* KO mice displayed very weak muscle force compared to WT mice, and all myotubularin constructs including MTMR2-L improved the TA specific muscle force (Fig. 3C). Noteworthy, a similar rescue was observed for MTM1 and MTMR2-S, significantly above that observed for MTMR2-L injected muscles. These results show that both MTMR2-L and MTMR2-S isoforms improve the muscle weakness due to loss of MTM1, and MTMR2-S expression induces a rescue akin to that observed by MTM1 gene replacement.

### The MTMR2 isoforms rescue the histopathological hallmarks of the Mtm1 KO mouse.

In the *Mtml KO* mice, TA injections of AAV2/1 carrying MTM1, MTMR2-L or MTMR2-S increased muscle mass (except for MTMR2-L) and force (Fig. 3). To analyze the rescue at the histological level, fiber size and nuclei localization were determined (Fig. 4). HE (hematoxylin-eosin) staining revealed increased fiber size in AAV-MTM1 and AAV-MTMR2-S than in *Mtml* KO muscle treated with empty AAV or MTMR2-L (Fig. 4A), even though we observed spatial heterogeneity in the muscle, with some regions still displaying smaller atrophic fibers. Morphometric analysis revealed that among the different myotubularins tested, MTM1 induced a clear shift toward larger fiber diameters compared to MTMR2 constructs and empty AAV (Fig. 4C). A very significant difference (P<0.0001) was observed between AAV-MTM1 (mean 58.4%) and AAV-MTMR2-L (mean 26.2%) in the percentage of muscle fibers having an area above 800 µm², and the difference was less significant (P=0.033) between MTM1 and MTMR2-S (39.8%) (Fig. 4D). Since nuclei are abnormally located within muscle fibers in *Mtml* KO mice, the distribution of nuclei was analyzed. Injection of MTM1, MTMR2-S or MTMR2-L into the TA muscle of *Mtm1* KO increased significantly the percentage of well-positioned peripheral nuclei compared with contralateral control muscles injected with empty AAV (Fig. 4E). The succinate dehydrogenase (SDH) staining shows accumulation at the periphery and center in the *Mtml* KO fibers (Amoasii, L., Bertazzi, D.L., Tronchere, H., Hnia, K., Chicanne, G., Rinaldi, B., Cowling, B.S., Ferry, A., Klaholz, B., Payrastre, B. et al. (2012) PLoS Genet, 8, e1002965), while it is greatly ameliorated upon expression of the different myotubularin constructs (Fig. 4B). These results show that both MTMR2 isoforms were able to ameliorate the histopathological hallmarks of the MTM1 myopathy, where MTMR2-S was more effective.

### MTMR2 isoforms rescue Mtm1 KO muscle disorganization and normalize PtdIns3P levels.

Patients with myotubular myopathy and the *Mtml* KO mice display an intracellular disorganization of their muscle fibers at the ultrastructural level (Buj-Bello, 2002 ; Spiro, A.J., Shy, G.M. and Gonatas, N.K. (1966) Myotubular myopathy. Persistence of fetal muscle in an adolescent boy. Arch. Neurol., 14, 1-14). To determine the organization of the contractile apparatus and triads, the ultrastructure of the different injected TA muscles was assessed by electron microscopy. As previously published, it was observed Z-line and mitochondria misalignment, thinner sarcomeres and lack of well-organized triads in the *Mtml* KO muscle injected with empty AAV (Amoasii, L., Bertazzi, D.L., Tronchere, H., Hnia, K., Chicanne, G., Rinaldi, B., Cowling, B.S., Ferry, A., Klaholz, B., Payrastre, B. et al. (2012) PLoS Genet, 8, e1002965) (Fig. 5A). Expression of MTM1 and both MTMR2 isoforms improved these different phenotypes, with the observation of well-organized triads with two sarcoplasmic reticulum cisternae associated with a central transverse-tubule (T-tubule) in muscles injected with MTM1, MTMR2-L or MTMR2-S (Fig. 5A). Moreover, AAV-mediated expression of MTM1, MTMR2-L and MTMR2-S increased the number of triads per sarcomere back to almost WT levels, with a better effect for MTMR2-S compared to MTMR2-L (Fig. 5B).

In yeast, only MTMR2-S but not MTMR2-L regulated the PtdIns3P myotubularin substrate level, as well as the one of PtdIns(3,5)*P₂* as assessed by vacuolar morphology (Fig. 2B). To determine whether the rescuing capacity of MTMR2 in mice was linked to its enzymatic activity, we quantified the intracellular levels of PtdIns3*P* in the AAV empty, MTM1, MTMR2-L and MTMR2-S injected TA muscles of *Mtm1* KO mice (Fig. 6A). PtdIns3*P* level was 2.3 fold higher in empty AAV injected *Mtm1* KO muscle than in WT muscle, reflecting the impact of the loss of MTM1 on its PtdIns3*P* lipid substrate. Upon expression of MTM1, the PtdIns3*P* level decreased to wild type levels, reflecting the *in vivo* phosphatase activity of MTM1. Both MTMR2 isoforms induced a decrease in PtdIns3*P* level when expressed in the *Mtml* KO mice, however only the short MTMR2-S isoform normalized PtdIns3*P* to wild type levels. These results show that MTMR2 displays an *in vivo* enzymatic activity in muscle. Moreover, the MTMR2 catalytic activity correlates with the rescue observed by exogenous expression in the *Mtml* KO myopathic mice.

Taken together, the results in *Mtml* KO mice expressing MTM1 or MTMR2 isoforms show that the different phenotypes associated to the myopathy including reduced muscle force, myo fiber atrophy, nuclei mispositioning, sarcomere and triad disorganization and increased PtdIns3*P* levels, were ameliorated compared to the control muscle injected with empty AAV (Table 1). Noteworthy, as observed in yeast studies, the shorter isoform MTMR2-S provided a better rescue than MTMR2-L, and was often comparable to MTM1.

**Table 1: Rescuing effects of MTM1 and MTMR2 isoforms on several hallmarks of myotubular myopathy.**

| | *Mtm1* KO + empty AAV | *Mtm1* KO + MTM1 | *Mtm1* KO + MTMR2-L | *Mtm1* KO + MTMR2-S | WT + empty AAV |
|---|---|---|---|---|---|
| Muscle weight | - | ++ | - | ++ | +++ |
| Muscle force | - | ++ | + | ++ | +++ |
| Fiber size | - | ++ | + | + | +++ |
| Nuclei positioning | - | ++ | ++ | ++ | +++ |
| Number of well-organized triads/sarcomere | - | ++ | + | ++ | +++ |
| PtdIns*3P* level | - | +++ | ++ | +++ | +++ |

| | | | | | |
|---|---|---|---|---|---|
| "+,++,+++": increasing rescuing ability of myotubularins, ranging from "-": no rescue to "+++": WT phenotype | | | | | |

### Expression of the MTMR2 short isoform is reduced in the Mtm1 KO mice muscles

Based on the GTEx expression database, the different MTMR2 mRNA variants (V1 to V4) producing these two MTMR2 protein isoforms are expressed in different tissues, with a low expression level in the skeletal muscle (Fig. 1). However, despite their strong rescue properties upon overexpression in TA muscles *of Mtml* KO mice (Fig. 3-5, 6A; Table 1), endogenous expression of MTMR2 variants does not compensate for the loss of MTM1 function in the myopathy patients. To help understand the difference in rescue observed between the MTMR2-Land -S isoforms, we quantified mRNA levels of the different MTMR2 variants (V1 to V4) in TA muscles of *Mtm*1 KO compared to wild type (WT) mice (Fig. 6B). The results show that MTMR2 mRNA total level was decreased in *Mtm1* KO muscles by 2 fold. This was mainly due to a strong decrease in the V2 and V3 transcripts encoding the MTMR2-S isoform, while the level of the V1 transcript coding for MTMR2-L remained statistically unchanged between *Mtml* KO and WT mice. Note that these decrease were not observed in Fig. 1B since it presents a conventional RT-PCR that does not allow quantification. As similar downregulation of V2 and V3 transcripts encoding the MTMR2-S isoform was observed in XLCNM patient muscles (Fig. 6C). These data suggest that the lack of compensation of MTM1 loss by endogenous MTMR2 is linked to the low expression level of MTMR2 associated to MTMR2-S decreased level in skeletal muscles. Alternatively, this could be linked to the low level of MTMR2 proteins in the muscle.

### Discussion

Here it was aimed to determine functional specificities and redundancies of MTM1 and MTMR2 myotubularins belonging to the same family of proteins, but whose mutations result in different diseases affecting different tissues, a myopathy and a neuropathy, respectively.

Their abilities to compensate for each other as a potential novel therapeutic strategy were also tested. Using molecular investigations and overexpression of these human myotubularins in yeast cells and in the skeletal muscle of the *Mtml* KO myopathic mice, it was characterized two MTMR2 isoforms with different catalytic activities linked to their ability to access their PPIn substrates. Moreover, it was showed that overexpression of MTMR2 rescues the myopathy due to MTM1 loss and that compared to MTMR2-L, the short MTMR2-S isoform displayed a better PtdIns3*P* phosphatase activity in yeast and in mice, correlating with better rescuing properties in myotubularin-depleted *ymr1Δ* yeast cells and in *Mtml* KO mice. The fact that MTMR2-L partially improved the phenotypes *of Mtml* KO mice despite performing poorly in yeast assays could be due the a lack of regulatory proteins in the yeast heterologous system.

### MTMR2 isoforms and functions

There are four naturally occurring MTMR2 mRNA variants in human and mice encoding two protein isoforms (MTMR2-L and -S), differing by a 72 aa extension at the N-terminal. MTMR2-S displayed a higher phosphatase activity than MTMR2-L *in vivo* in yeast and mouse, suggesting the N-terminal is important for the regulation of MTMR2 function. The phosphorylation of the serine 58, within this N-terminal extension, was shown to be important for MTMR2 endosomal membrane localization and catalytic function. Indeed, the MTMR2-S58A phosphorylation-deficient mutant was localized to membrane structures and was active towards PtdIns3*P*, contrary to the phosphomimetic mutant MTMR2-S58E. Here, it is shown that the MTMR2-S protein lacking the N-terminal sequence encompassing the S58 phosphorylated residue is concentrated to membranes when expressed in yeast (Fig. 2B) and is more active towards PtdIns3P compared to MTMR2-L in yeast (Fig. 2D) and in murine muscles (Fig. 6A). The N-terminal extension of MTMR2 was not resolved in the crystallographic structure, supporting the hypothesis that it can adopt different conformations and might regulate MTMR2 functions (Begley, M.J., Taylor, G.S., Brock, M.A., Ghosh, P., Woods, V.L. and Dixon, J.E. (2006) Molecular basis for substrate recognition by MTMR2, a myotubularin family phosphoinositide phosphatase. Proc. Natl. Acad. Sci. U. S. A., 103, 927-932 ; Begley, M.J., Taylor, G.S., Kim, S.A., Veine, D.M., Dixon, J.E. and Stuckey, J.A. (2003) Crystal structure of a phosphoinositide phosphatase, MTMR2: insights into myotubular myopathy and Charcot-Marie-Tooth syndrome. Mol. Cell, 12, 1391-1402). These results show that there are two forms of MTMR2, MTMR2-S mainly membrane localized and with high phosphatase activity *in vivo* and MTMR2-L whose membrane localization is dependent on phosphorylation at the S58 residue. Interestingly, in brain expression is biased towards the *MTMR2* V1 variant coding for MTMR2-L (Fig. 1). The S58 phosphorylation is mediated by Erk2 kinase whose expression in brain is precisely higher than in other tissues, correlating with MTMR2-L expression (GTEx database).

### Functional redundancy and compensation within myotubularins

There are 14 myotubularins mostly ubiquitously expressed in human tissues, but the loss of MTM1 leads specifically to a severe congenital myopathy. This reveals that *MTM1* homologs, notably the closer *MTMR2* homolog, do not compensate for the lack of MTM1 in the skeletal muscles when expressed at endogenous levels. Here it is evidenced that MTMR2-S is downregulated in the skeletal muscles of the myopathic *Mtml* KO mice. Moreover, compared to brain and other tissues, the expression of MTMR2 transcripts is low in skeletal muscles. Altogether this suggests that this low expression of MTMR2 in muscle exacerbated by its downregulation in the myopathy mouse model and in XLCNM patient muscles is the basis for the lack of compensation. Indeed, the MTMR2-S improves better both functional and structural myopathic phenotypes and is more significantly downregulated than MTMR2-L in the myopathic muscles. This reveals that the molecular basis for the functional difference between MTM1 and MTMR2 resides in the N-terminal extension upstream the PH-GRAM domain, with the MTMR2-S lacking this extension displaying similar *in vivo* functions as MTM1 in yeast and in mice. Removal of this N-terminal extension in the native MTMR2-L isoform converts MTMR2 activity into an MTM1-like activity.

### MTMR2-S as a novel therapeutic target for myotubular myopathy

MTMR2-S could thus be used as a therapeutic target. Intramuscular AAV transduction of human MTMR2-S into *Mtml* KO mice greatly improved the phenotypes, supporting the rescue is cell autonomous in muscle. While this actual protocol aimed to investigate the cell autonomous compensation by MTMR2 through intramuscular injection, it was not possible to determine the extent of the rescue and the long-term potential of MTMR2-mediated rescue as *Mtml* KO mice die at around 2 months most probably from respiratory failure and feeding defect. These data support that MTMR2-S isoform has a better rescuing ability than the main described MTMR2-L isoform and is a naturally occurring variant, including in muscle. Since MTMR2-S transcripts are decreased in the *Mtml* KO muscles, a potential strategy will be to promote their expression by modulation of *MTMR2* alternative splicing or exogenous expression.

### Effect of MTMR2-S expression on the overall mouse through systemic injections

As shown above, intramuscular injections allowed to investigate the muscle-specific functions and rescuing capacities of MTM1 and MTMR2 in the *Mtml* KO mouse model. To complete this study and observe the effect of MTMR2 expression on the overall mouse, systemic injections were performed.

Wild type or *Mtml* KO pups were intraperitoneally injected at birth or at Day 1 by 1.5×10¹² units of empty AAV viral particles or AAV overexpressing human MTM1 or MTMR2-S. Then 3 weeks after injection the body weight and the mice skeletal muscle strength were measured by two different tests: the grip test and the hanging test. Mice were sacrificed from 7 weeks of age when Mtm1 KO affected mice injected with empty AAV were still alive, allowing to compare the myotubularin overexpression to the empty vector.

Overexpression of MTM1 and MTMR2-S isoforms were assessed by western blot on tibialis anterior and diaphragm skeletal muscles. In both cases, myotubularins were well detected at the expected size, as seen above for intramuscular injections. This confirmed that all myotubularins were well expressed in skeletal muscles after systemic delivery of AAV at day 1 postnatally in mice.

The effect of systemic expression of the myotubularins on the lifespan and the body weight of the injected mice was analyzed. The first major observation was that MTMR2-S increased the lifespan of Mtm1 KO mice (3/4 survived until weeks 7-10). While the myopathic mice usually die around 5-7 weeks of age, the MTMR2-S overexpression allowed two mice to reach 10 weeks-old (oldest timepoint measured). MTM1 was already published to have a similar rescuing effect on the lifespan. This experiment confirms the MTMR2-S isoform can increase the lifespan of *Mtm1* knockout mice.

Major clinical phenotypes of myopathic *Mtml* KO mice are the lower body weight since 2 to 3 weeks of age compared to WT mice, and the progressing loss of weight starting around 5 weeks of age (Cowling et al., 2014). The latter is mainly due to a loss of muscle mass and in the final steps of the disease to difficulties to reach their food. In contrast, WT mice continue to progressively gain weight during the first 10-12 weeks of their life (Figure 9). Mice overexpressing human MTM1 are initially bigger than *Mtml* KO mice injected with empty AAV, and perfectly gain body weight at the same rate than WT mice. In comparison, mice overexpressing MTMR2-S also show a good rescue of the body weight from 3 weeks to 5 weeks old, but then start to lose weight and reach the *Mtml* KO level (Figure 9). In correlation, the positive effects of MTMR2-S expression were clinically clear (but not quantified) until 5 weeks of age, then the mice started to progressively develop the *Mtml* KO typical phenotypes (loss of muscle weight and force, scoliosis, difficulties to breath and to walk).

These results show that MTMR2 short isoform improved the lifespan and body weight of *Mtm1* KO mice.

The other obvious clinical feature of myotubular myopathy is the severe muscle weakness that is reproduced in the *Mtml* KO mouse model. The hanging test measures whole body strength. Overexpression of MTMR2-S isoform allowed the *Mtml* KO mice to progressively hang longer (starting at 30-40 seconds), until they reach the WT level and were able after 7 weeks to hang for 3 x 60 seconds (Figure 10). At the same age, half of the *Mtml* KO mice are usually dead, and the two mice that were tested were not able to hang more than few seconds after 5 weeks. MTM1 effect was even better and allowed the Mtm1 KO mice to perfectly hang for 60 seconds after 4 weeks. No negative effect was observed for any myotubularin on WT mice that were always able to hang for 60 seconds.

These results showed that MTMR2-S isoform rescued the muscle strength *of Mtml* KO mice. Notably these mice injected with MTMR2 isoforms that were sick in appearance (difficulties to breath, scoliosis) could hang for 60 seconds as well as WT mice, suggesting a strong improvement in whole body strength (Figure 10).

### Conclusions

Intramuscular injections identified both short and long MTMR2 isoforms improved the myopathic phenotype, with the short isoform (MTMR2-S) inducing a better rescuing effect when compared side-by-side. Systemic injections confirmed MTMR2-S isoform expression is able to delay the myopathic phenotype onset in *Mtml* KO mice and significantly rescued their muscle force. Mice overexpressing MTMR2 isoforms were still affected but clearly more mobile than Mtm1 KO mice. Altogether, this systemic study shows satisfactory preliminary data, supporting the overexpression of MTMR2-S is able to improve the myopathic phenotype in *Mtml* knockout mice, a mouse model for Myotubular Myopathy.

### MTMR2-S Sequences

SEQ ID NO: 1 (human MTMR2-S protein)
   Human MTMR2-S protein sequence (NP_001230500.1, NP_958438.1 etNP_958435.1)
SEQ ID NO: 2 (nucleotide human MTMR2-S, cDNA) coding sequence
   Human MTMR2-S coding sequence (CCDS:CCDS8306.1 - NM_201278.2:664..2379)
3 isoforms RNA encoding for the same protein MTMR2-S:
   SEQ ID NO: 3: cDNA corresponding to Human MTMR2 mRNA V2 (NM_201278.2)
   SEQ ID NO: 4: cDNA corresponding to Human MTMR2 mRNA V3 (NM_201281.2)
   SEQ ID NO 5: cDNA corresponding to Human MTMR2 mRNA V4 (NM_001243571.1)

## Claims

1. A MTMR2-S polypeptide, or a nucleic acid sequence producing or encoding said MTMR2-S polypeptide, for use in the treatment of X-linked centronuclear myopathy (XLCNM).

2. The polypeptide or nucleic acid sequence for use according to claim 1, wherein it is to improve muscle function or increasing formation of muscle.

3. The polypeptide or nucleic acid sequence for use according to claim 1 or 2, wherein the polypeptide is selected from the group consisting of:
- a polypeptide which has an amino acid sequence at least 90% identical (or homologous) to SEQ ID NO: 1, or a bioactive fragment or variant thereof; and
- a polypeptide which comprises an amino acid sequence at least 80%, 85%, 90%, 95%, 97%, 98%, 99% or 100% identical to SEQ ID NO: 1 and which comprises 571 amino acids length or less, or a bioactive fragment or variant thereof.

4. The polypeptide or nucleic acid sequence for use according to any one of claims 1 to 3, wherein the nucleic acid sequence is a naked nucleic acid sequence or is within a construct useful for producing such polypeptide or a vector comprising the same.

5. The polypeptide or nucleic acid sequence for use according to claim 4, wherein the nucleic acid sequence comprises a sequence comprising at least one of SEQ ID NOs: 2, 3, 4 or 5.

6. The polypeptide or nucleic acid sequence for use according to any one of the preceding claims, wherein the polypeptide or the nucleic acid sequence are comprised in a composition, preferably in a pharmaceutically acceptable support.

7. The polypeptide or nucleic acid sequence for use according to any one of claims 1 to 6, wherein the nucleic acid sequence is comprised in a nucleic acid construct, recombinant expression vector, or recombinant host cell and is operably linked to one or more control sequences that direct the production of the MTMR2-S polypeptide.

## Patentansprüche

1. Ein MTMR2-S-Polypeptid oder eine Nukleinsäuresequenz, die dieses MTMR2-S-Polypeptid produziert oder enkodiert, zur Verwendung bei der Behandlung von X-gebundener zentronukleärer Myopathie (XLCNM).

2. Polypeptid- oder Nukleinsäuresequenz zur Verwendung nach Anspruch 1, wobei sie zur Verbesserung der Muskelfunktion oder Steigerung der Muskelbildung dient.

3. Polypeptid oder Nukleinsäuresequenz zur Verwendung nach Anspruch 1 oder 2, wobei das Polypeptid ausgewählt ist aus der Gruppe bestehend aus:
- einem Polypeptid, das eine Aminosäuresequenz aufweist, die zu mindestens 90 % mit SEQ ID NO: 1 identisch (oder homolog) ist, oder ein bioaktives Fragment oder eine Variante davon; und
- einem Polypeptid, das eine Aminosäuresequenz umfasst, die zu mindestens 80 %, 85 %, 90 %, 95 %, 97 %, 98 %, 99 % oder 100 % mit SEQ ID NO: 1 identisch ist, und das eine Länge von 571 Aminosäuren oder weniger umfasst oder ein bioaktives Fragment oder eine Variante davon.

4. Polypeptid oder Nukleinsäuresequenz zur Verwendung nach einem der Ansprüche 1 bis 3, wobei die Nukleinsäuresequenz eine nackte Nukleinsäuresequenz ist oder sich innerhalb eines Konstrukts befindet, das zur Herstellung eines solchen Polypeptids oder eines Vektors, der dasselbe umfasst, verwendbar ist.

5. Polypeptid- oder Nukleinsäuresequenz zur Verwendung nach Anspruch 4, wobei die Nukleinsäuresequenz eine Sequenz umfasst, die mindestens eine der SEQ ID NOs: 2, 3, 4 oder 5 umfasst.

6. Polypeptid- oder Nukleinsäuresequenz zur Verwendung nach einem der vorhergehenden Ansprüche, wobei das Polypeptid oder die Nukleinsäuresequenz in einer Zusammensetzung, vorzugsweise in einem pharmazeutisch verträglichen Träger, enthalten sind.

7. Polypeptid oder Nukleinsäuresequenz zur Verwendung nach einem der Ansprüche 1 bis 6, wobei die Nukleinsäuresequenz in einem Nukleinsäurekonstrukt, einem rekombinanten Expressionsvektor oder einer rekombinanten Wirtszelle enthalten ist und operabel mit einer oder mehreren Kontroll-Sequenzen verbunden ist, die die Produktion des MTMR2-S-Polypeptids steuern.

## Revendications

1. Polypeptide MTMR2-S, ou une séquence d'acide nucléique produisant ou encodant ledit polypeptide MTMR2-S, pour utilisation dans le traitement d'une myopathie centronucléaire liée à l'X (XLCNM).

2. Polypeptide ou séquence d'acide nucléique pour utilisation selon la revendication 1, qui est destiné à améliorer la fonction musculaire ou augmenter la formation de muscle.

3. Polypeptide ou séquence d'acide nucléique pour utilisation selon la revendication 1 ou 2, dans lequel le polypeptide est choisi dans le groupe constitué par :
- un polypeptide qui a une séquence d'acides aminés identique à au moins 90 % (ou homologue) à la SEQ ID NO : 1, ou un variant ou fragment bioactif de celui-ci ; et
- un polypeptide qui comprend une séquence d'acides aminés identique à au moins 80 %, 85 %, 90 %, 95 %, 97 %, 98 %, 99 % ou 100 % à la SEQ ID NO : 1, et qui comprend une longueur de 571 acides aminés ou moins ou un variant ou fragment bioactif de celui-ci.

4. Polypeptide ou séquence d'acide nucléique pour utilisation selon l'une quelconque des revendications 1 à 3, dans lequel la séquence d'acide nucléique est une séquence d'acide nucléique nue ou est à l'intérieur d'un produit de construction utile pour produire ce polypeptide ou un vecteur le comprenant.

5. Polypeptide ou séquence d'acide nucléique pour utilisation selon la revendication 4, dans lequel la séquence d'acide nucléique comprend une séquence comprenant au moins l'une des SEQ ID NO : 2, 3, 4 ou 5.

6. Polypeptide ou séquence d'acide nucléique pour utilisation selon l'une quelconque des revendications précédentes, dans lequel le polypeptide ou la séquence d'acide nucléique est compris dans une composition, de préférence dans un support pharmaceutiquement acceptable.

7. Polypeptide ou séquence d'acide nucléique pour utilisation selon l'une quelconque des revendications 1 à 6, dans lequel la séquence d'acide nucléique est comprise dans un produit de construction d'acide nucléique, un vecteur d'expression recombiné, ou une cellule hôte recombinée, et est lié de manière opérationnelle à une ou plusieurs séquences de contrôle qui dirigent la production du polypeptide MTMR2-S.
